# EUROPEAN PATENT SPECIFICATION

(11) **EP 3 051 291 B1**
(45) Date of publication and mention of the grant of the patent: **30.01.2019**
(21) Application number: 14796149.4
(22) Date of filing: 26.09.2014
(51) Int. Cl.: G01N 33/574

(54) **METHOD FOR THE DIAGNOSIS AND PROGNOSIS OF SKIN MELANOMA**
DIAGNOSTISCHES UND PROGNOSTISCHES VERFAHREN FÜR HAUT-MELANOMA
MÉTHODE DIAGNOSTIQUE ET PRONOSTIQUE DU MÉLANOME DE LA PEAU

(30) Priority: 26.09.2013 ES 201331404
(43) Date of publication of application: 03.08.2016
(73) Proprietor: Universidad del Pais Vasco, 48940 Leioa Vizcaya (ES); Administración General De La Communidad Autónoma De Euskadi, 01010 Vitoria-Gasteiz (ES)
(72) Inventor: BOYANO LÓPEZ, María Dolores, 48940 Leioa - Vizcaya (ES); ARROLLO BERDUGO, Yoana, 48940 Leioa - Vizcaya (ES); ALONSO ALEGRE, Santos, 48940 Leioa - Vizcaya (ES); ASUMENDI MALLEA, Aintzane, 48940 Leioa - Vizcaya (ES); PÉREZ-YARZA PÉREZ-IREZABAL, Gorka, 48940 Leioa - Vizcaya (ES); CAREAGA ALZAGA, Jesús María, 48013 Bilbao - Vizcaya (ES); GARDEAZABAL GARCÍA, Jesús, 48903 Barakaldo - Vizcaya (ES); IZU BELLOSO, Rosa, 48013 Bilbao - Vizcaya (ES); DÍAZ RAMÓN, José Luis, 48903 Barakaldo - Vizcaya (ES); ACEBO MARIÑAS, Elvira, 48903 Barakaldo - Vizcaya (ES); SÁNCHEZ DÍEZ, Ana, 48013 Bilbao - Vizcaya (ES); MARTÍNEZ DELIZARDUY, Íñigo, 48013 Bilbao - Vizcaya (ES); RATÓN NIETO, Juan Antonio, 48903 Vizcaya (ES)
(74) Representative: ABG Intellectual Property, S.L.
(86) International application number: PCT/ES2014/070737
(87) International publication number: WO 2015/044499

(56) References cited:
- LICCIULLI SILVIA ET AL: "Pirin delocalization in melanoma progression identified by high content immuno-detection based approaches", BMC CELL BIOLOGY, BIOMED CENTRAL, LONDON, GB, vol. 11, no. 1, 20 January 2010 (2010-01-20), pages 1-8, XP021065794, London ISSN: 1471-2121
- P VEREECKEN ET AL: "A synopsis of serum biomarkers in cutaneous melanoma patients", DERMATOLOGY RESEARCH AND PRACTICE, vol. 2012, 2 January 2012 (2012-01-02), pages 1-7, XP055168518,

### SEQUENCE LISTING

<110> Universidad del País Vasco Administración General de la Comunidad Autónoma de Euskadi
<120> Método de diagnóstico y pronóstico de melanoma cutáneo
<130> P10196PC00
<150> ESP201331404
   <151> 20130926
<160> 16
<170> PatentIn version 3.5
<210> 1
   <211> 23
   <212> DNA
   <213> Artificial Sequence
<220>
   <223> Cebador directo beta actina
<400> 1
   agatgaccca gatcatgttt gag 23
<210> 2
   <211> 19
   <212> DNA
   <213> Artificial Sequence
<220>
   <223> Cebador reverso beta actina
<400> 2
   gtcaccggag tccatcacg 19
<210> 3
   <211> 19
   <212> DNA
   <213> Artificial Sequence
<220>
   <223> Cebador directo GAPDH
<400> 3
   cctgttcgac agtcagccg 19
<210> 4
   <211> 20
   <212> DNA
   <213> Artificial Sequence
<220>
   <223> Cebador reverso GAPDH
<400> 4
   cgaccaaatc cgttgactcc 20
<210> 5
   <211> 19
   <212> DNA
   <213> Artificial Sequence
<220>
   <223> Cebador directo Rig/S15
<400> 5
   ttccgcaagt tcacctacc 19
<210> 6
   <211> 20
   <212> DNA
   <213> Artificial Sequence
<220>
   <223> Cebador reverso Rig/S15
<400> 6
   cgggccggcc atgctttacg 20
<210> 7
   <211> 19
   <212> DNA
   <213> Artificial Sequence
<220>
   <223> Cebador directo BCL2
<400> 7
   ggggaggatt gtggccttc 19
<210> 8
   <211> 19
   <212> DNA
   <213> Artificial Sequence
<220>
   <223> Cebador reverso BCL2
<400> 8
   cagggcgatg ttgtccacc 19
<210> 9
   <211> 22
   <212> DNA
   <213> Artificial Sequence
<220>
   <223> Cebador directo BCL3
<400> 9
   aacctgccta cacccctata cc 22
<210> 10
   <211> 21
   <212> DNA
   <213> Artificial Sequence
<220>
   <223> Cebador reverso BCL3
<400> 10
   gcaccacagc aatatggaga g 21
<210> 11
   <211> 20
   <212> DNA
   <213> Artificial Sequence
<220>
   <223> Cebador directo M-MITF
<400> 11
   ctcgaaaacc ccaccaagta 20
<210> 12
   <211> 20
   <212> DNA
   <213> Artificial Sequence
<220>
   <223> Cebador reverso M-MITF
<400> 12
   gacatggcaa gctcaggact 20
<210> 13
   <211> 21
   <212> DNA
   <213> Artificial Sequence
<220>
   <223> Cebador directo PEBP1
<400> 13
   aatagaccca ccagcatttc g 21
<210> 14
   <211> 21
   <212> DNA
   <213> Artificial Sequence
<220>
   <223> Cebador reverso PEBP1
<400> 14
   gtgccactgc tgatgtcatt g 21
<210> 15
   <211> 21
   <212> DNA
   <213> Artificial Sequence
<220>
   <223> Cebador directo PIR
<400> 15
   ggagcctcag taccaggaac t 21
<210> 16
   <211> 21
   <212> DNA
   <213> Artificial Sequence
<220>
   <223> Cebador reverso PIR
<400> 16
   cttggacttt attcccaggg c 21

## Description

### Technical Field of the Invention

The present invention is generally encompassed in the field for the diagnosis and prognosis of diseases; particularly, for the diagnosis and prognosis of cutaneous melanoma and for the differential diagnosis of cutaneous melanoma and a non-tumoral cutaneous lesion.

### Background of the Invention

Malignant melanoma is the main cause of skin cancer-associated mortality with increasing incidence in recent years, particularly among white fair-skinned people, with 160,000 new cases being diagnosed annually worldwide.

Molecular alterations involved in the pathogenesis of malignant melanoma are constantly studied, which has allowed determining genetic alterations affecting DNA repair genes, oncogenes and tumor suppressor genes, in addition to genes involved in the pigmentation pathways. However, these progresses are still insufficient to establish biological markers and molecular targets making early diagnosis easier and allowing the development of more effective therapies for this neoplasm.

Several melanoma tumor markers are known and they include serum LDH, 5-S-CD, S-100β and MIA (Garbe C. et al., Cancer. Apr 1, 2003;97(7):1737-45). However, none of these tumor markers gives positive results unless the melanoma is in a very advanced stage, such as stage IV.

Vereecken et al. (Vereecken et al. 2012. Dermatology Research and Practice, vol. 2012: 1-7) disclose several serum biomarkers in cutaneous melanoma patients. However, the poor sensitivity and specificity of those markers are serious limitations for their routine use.

The primary tumor of a malignant melanoma has a biological behavior where two growth phases are distinguished. The first phase is referred to as radial growth phase (RGP) because the melanocytes proliferate intraepidermally. The RGP can last for months or years until the tumor acquires the capacity to invade the dermis, thus starting the so-called vertical growth phase (VGP). The VGP involves a poor prognosis given that the infiltration of neoplastic cells into the lower skin layers enables them to spread through the lymph vessels to the lymph nodes or, through the blood vessels, to any organ where the tumor cells can successfully proliferate, generating metastasis.

Today, the possibility of curing melanoma is closely linked to the timely diagnosis and surgical removal of the tumor. Therefore, the prognosis of the disease today is based fundamentally on the histological evaluation of biopsies and the size of the cutaneous lesions. About 70% of melanoma patients are diagnosed in the early stages of the disease (stage I and stage II according to the AJCC classification; Melanoma Staging Database, 2008), and they are considered "low risk" patients due to the high probability of being cured by means of surgery. However, for the cases of metastatic melanoma there are still no effective diagnostic methods or therapies capable of improving patient survival.

Melanoma patients' probability of survival is related to the absence of nodal and metastatic involvement. Sentinel node testing (which consists of detecting tumor cells in the lymph node chain closest to the tumor) has become a powerful prognosis marker for determining the survival of patients with malignant cutaneous melanoma with lesions between 1 and 4 mm in size.

The mortality detected in the early stages of tumor progression may be at least partially due to the fact that patients with lesions smaller than 1 mm are not subjected to sentinel node biopsy. On the other hand, a negative result in the sentinel node biopsy does not exclude the possibility of developing metastasis. In fact, about 10% of patients with negative sentinel node develop metastasis.

The only prognosis markers in clinical use to date are those included in the AJCC (American Join Committee of Cancer) staging system which is based on the TNM (tumor, affected lymph nodes and metastasis) classification.

Licciulli S. et al., (Licciulli S. et al. 2010. BMC Cell Biology, vol. 11(1): 1-8) describes that cytoplasmic delocalization of PIR and high expression levels of PIR are characteristic of advanced stages of a subset of cutaneous melanoma.

Today and unlike other tumors such as colon carcinoma or breast and lung carcinoma, no molecular marker whatsoever has been demonstrated to be useful in clinical practice since they do not allow predicting the risk in early stages (AJCC stage I to III).

There is therefore a need to develop new methods for the diagnosis of melanoma and methods for determining the probability that a subject with melanoma will develop metastasis, such that an additional treatment other than the surgical removal of the tumor can be applied, in the event that the tumor cells have already acquired the invasive phenotype.

### Brief Description of the Invention

In a first aspect, the invention relates to an *in vitro* method for diagnosing cutaneous melanoma which comprises:
a) determining the expression level of *PIR (Pirin)* in a sample from a subject, and
b) comparing the level obtained in step a) with a reference value for said marker,
wherein a decreased expression level of *PIR* with respect to the reference value for said marker is indicative that said subject suffers from cutaneous melanoma, wherein the sample is a tissue sample.

In another aspect, the invention relates to an *in vitro* method for determining the probability of metastasis in a subject diagnosed with cutaneous melanoma which comprises:
a) determining the expression level of *PIR* in a sample obtained from a subject, and
b) comparing the level obtained in step a) with a reference value for said marker,
wherein a decreased expression level of *PIR* with respect to the reference value for said marker is indicative that said subject has a high probability of developing metastasis, wherein the sample is a tissue sample.

In another aspect, the invention relates to an *in vitro* method for differentiating benign nevus from cutaneous melanoma in a cutaneous lesion which comprises:
a) determining the expression level of *PIR* in a tissue sample of said cutaneous lesion, and
b) comparing the level obtained in step a) with a reference value for said marker,
wherein a decreased expression level of *PIR* with respect to the reference value for said marker is indicative that said cutaneous lesion is a melanoma.

In another aspect, the invention relates to a kit comprising a reagent capable of determining the expression level of *PIR* further comprising a reagent capable of determining the expression level of a marker selected from the group consisting of *BCL3, PEBP1, M-MITF, BCL2* and combinations thereof, wherein the reagent capable of determining the expression level is an antibody or a nucleic acid.

In another aspect, the invention relates to the use of a kit comprising an antibody or a nucleic acid capable of determining the expression level of *PIR* or a kit of the invention for diagnosing cutaneous melanoma, for determining the probability of metastasis in a subject diagnosed with cutaneous melanoma or for differentiating a benign nevus from cutaneous melanoma.

### Brief Description of the Drawings

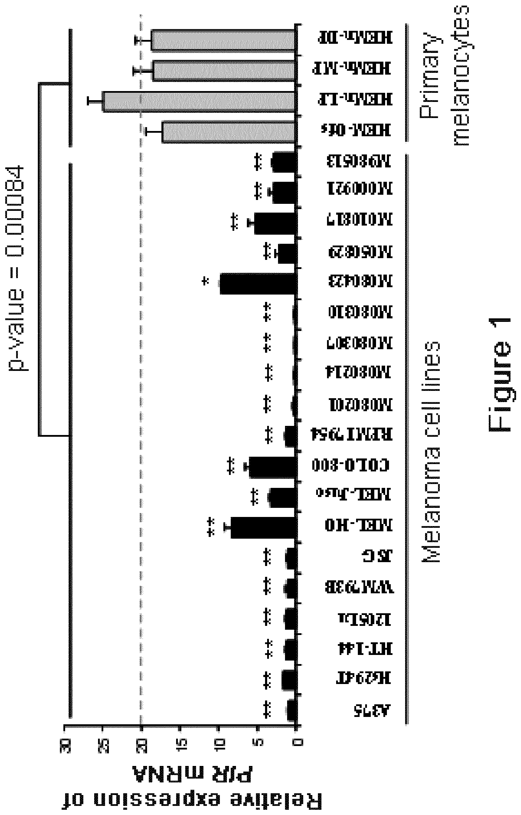

Figure 1 shows the relative expression of the *PIR* gene in melanoma lines and cultures of primary melanocytes. All the melanoma lines analyzed show a significant reduction in the expression levels of *PIR* with respect to the cultures of primary melanocytes. The mean expression of *PIR* in the cultures of primary melanocytes is 134 and it is indicated in the graph with a dotted line. The percentage (%) of expression is at least 75% less with respect to the melanocytes.

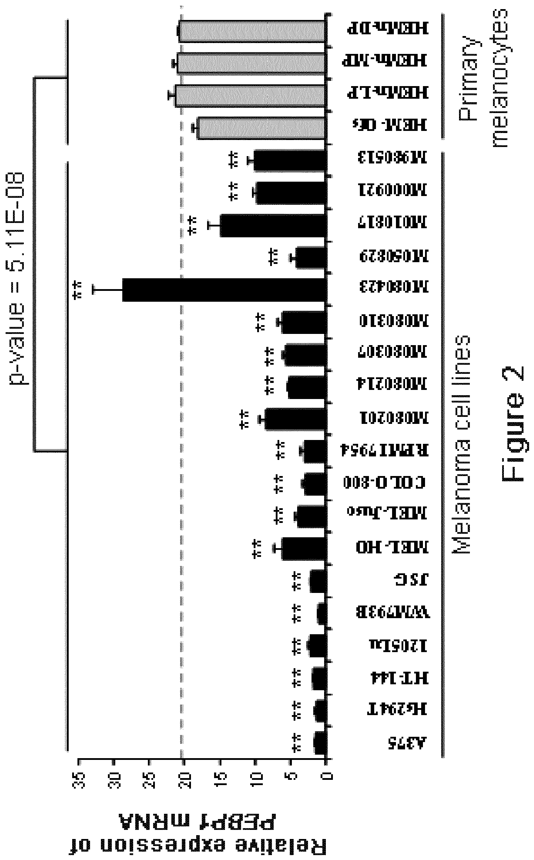

Figure 2 shows the relative expression of the *PEBP1* gene in melanoma cell lines and cultures of primary melanocytes. Gene expression quantification by means of RT-qPCR shows the silencing of the *PEBP1* gene in all the melanoma lines analyzed with respect to the cultures of primary melanocytes. The mean expression of *PEBP1* in cultures of primary melanocytes is 20.9 and it is indicated in the graph with a dotted line. The % of expression is at least 75% less with respect to the melanocytes.

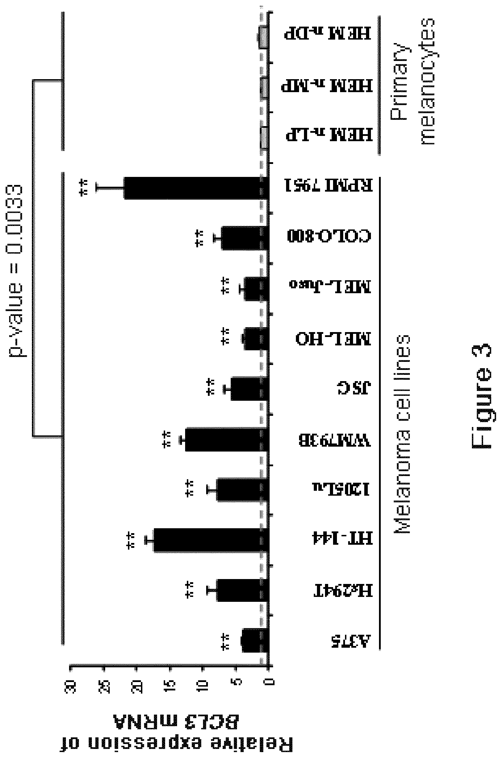

Figure 3 shows the relative expression of the *BCL3* gene in melanoma lines and cultures of primary melanocytes. The melanoma lines analyzed show a statistically significant overexpression of the *BCL3* gene in relation to the levels found in the cultures of primary melanocytes. The mean expression of *BCL3* in the cultures of primary melanocytes is 1.1 and it is indicated in the graph with a dotted line. The % of expression is at least 75% more than the melanocytes.

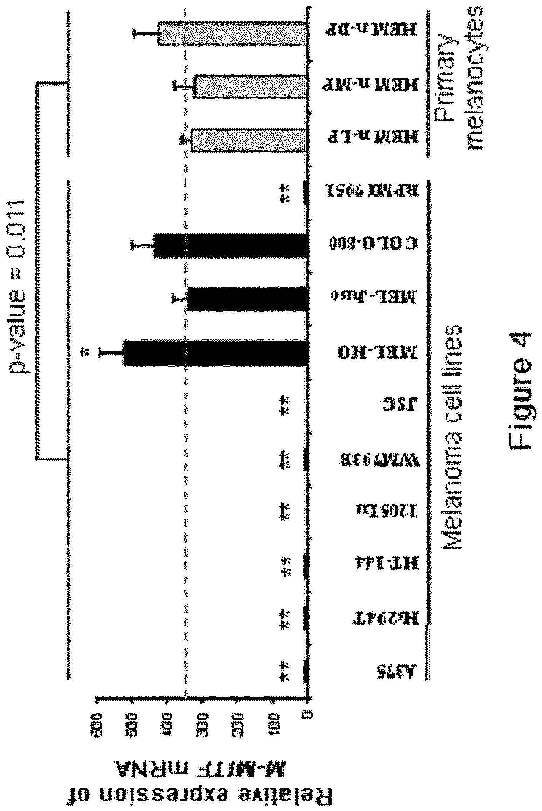

Figure 4 shows the relative expression of *M-MITF* in melanoma lines and cultures of primary melanocytes. The lines with invasive melanoma phenotype A375, Hs294T, HT-144, 1205Lu, WM793B, JSG and RPMI7951 show a significant reduction in *M-MITF,* whereas the lines with proliferative phenotype MEL-HO, MEL-Juso and COLO-800 have levels of mRNAs transcribed from this gene similar to those found in the cultures of primary melanocytes. The mean expression of *M-MITF* in the cultures of primary melanocytes is 356.1 and it is indicated in the graph with a dotted line. The % of expression in the invasive lines is at least 90% less with respect to normal melanocytes and non-invasive melanomas.

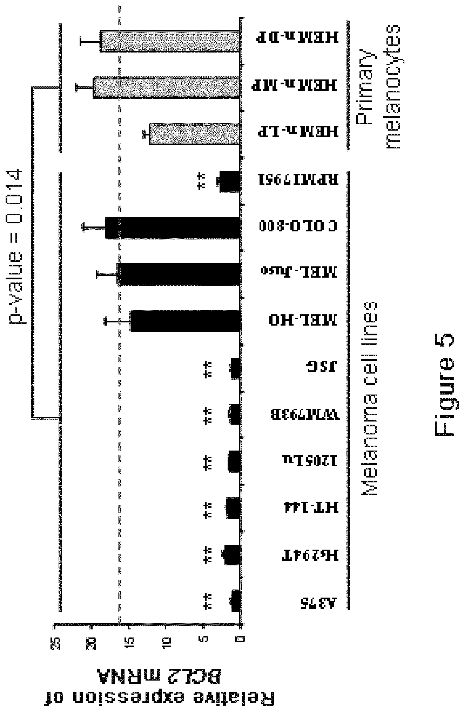

Figure 5 shows the relative expression of the *BCL2* gene in melanoma lines and cultures of primary melanocytes. The melanoma lines with invasive capacity A375, Hs294T, HT-144, 1205Lu, WM793B, JSG and RPMI7951 show a statistically significant reduction in the *BCL2* gene, whereas the non-invasive lines MEL-HO, MEL-Juso and COLO-800 have levels of mRNAs for this gene similar to those found in the cultures of primary melanocytes. The mean expression of the *BCL2* gene in the cultures of primary melanocytes is 16.8 and it is indicated in the graph with a dotted line. The % of expression in the invasive lines is at least 90% less with respect to normal melanocytes and non-invasive melanomas (indicated inside the box).

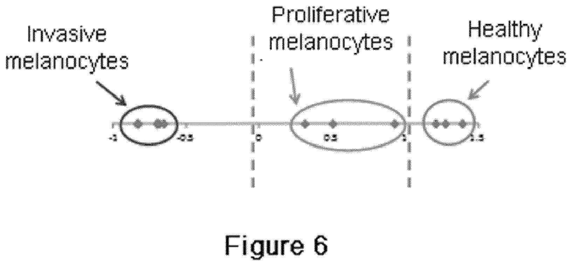

Figure 6 shows the distribution of the melanoma lines and the cultures of primary melanocytes analyzed in a component diagram by means of Varimax orthogonal rotation. For the analysis of the main components, the protein levels obtained and the gene expression levels obtained by means of RT-qPCR have been included.

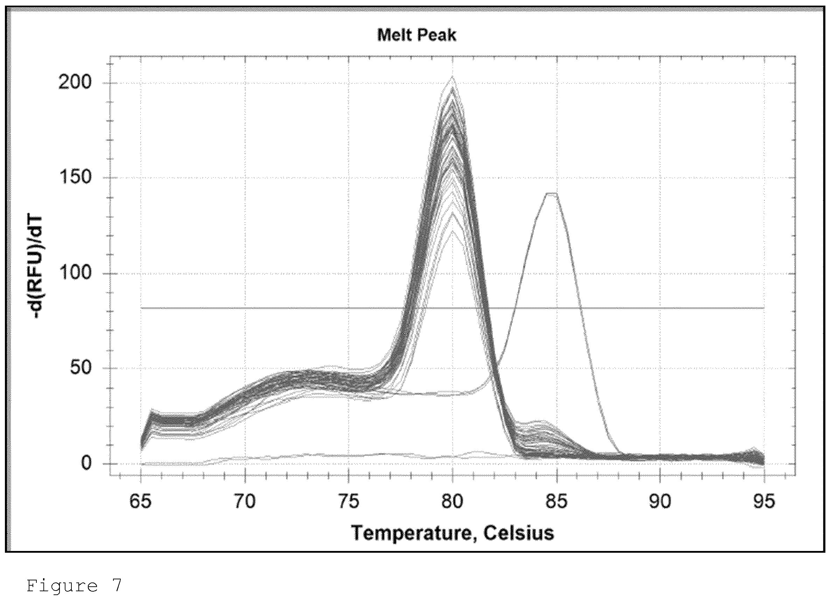

Figure 7 shows the melting curves of nevi and melanoma samples.

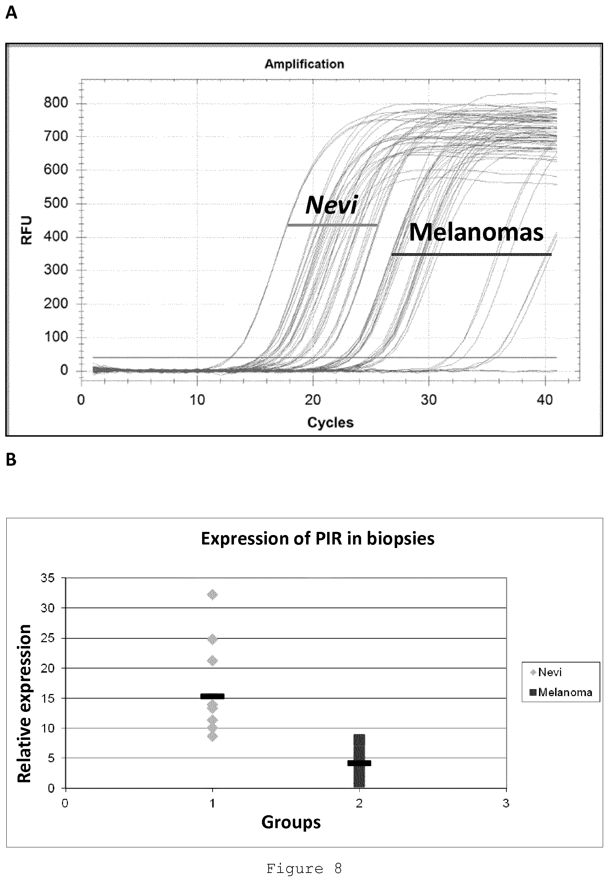

Figure 8 shows the amplification curves of all the melanoma and nevi samples (A). Comparison of the expression levels of PIR between melanoma and nevus samples (B). Nevi have a mean expression 3 times greater than the mean of melanomas (p<0.05).

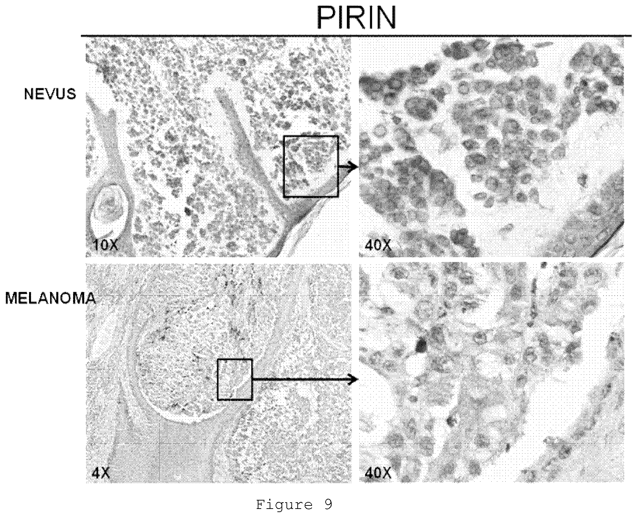

Figure 9 shows the expression of PIRIN in nevus and melanoma histological sections. Sections 6-10 µm thick were incubated with the anti-PIRIN monoclonal antibody and developed with AEC (EnVision G/2 System/AP, rabbit/mouse. Permanent Red, Dako). The nuclei were contrasted with hematoxylin.

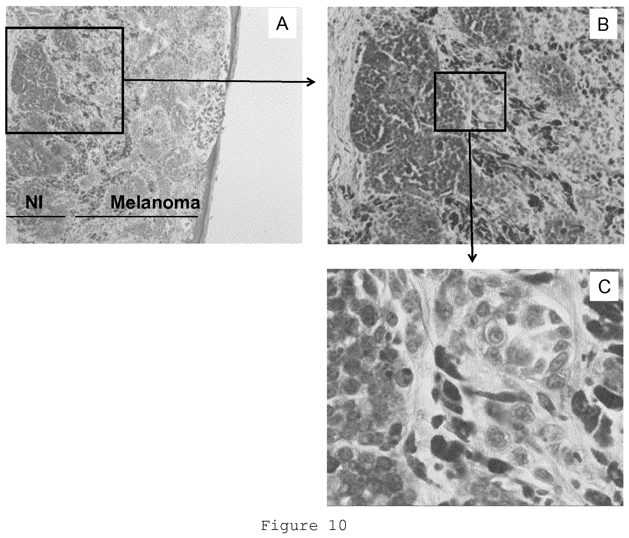

Figure 10 shows the expression of the Pirin protein in a histological section of a malignant melanoma on a stage III intradermal nevus (IN) according to AJCC. (A) 40x; (B) 200x; (C) 400x.

### Detailed Description of the Invention

The authors of the present invention have identified new markers, specifically *BCL3, PIR* and *PEBP1,* as markers for the diagnosis of cutaneous melanoma. They have also identified the reduction or absence of the expression of *PIR, M-MITF, BCL2* and combinations thereof as marker for the prognosis of cutaneous melanoma since it allows identifying cutaneous melanomas with invasive capacity.

### Methods of the Invention

In a first aspect the invention relates to an *in vitro* method (hereinafter, "first method") for diagnosing cutaneous melanoma which comprises:
a) determining the expression level of PIR (Pirin) in a sample from a subject, and
b) comparing the level obtained in step a) with a reference value for said marker,
wherein a decreased expression level of PIR with respect to the reference value for said marker is indicative that said subject suffers from cutaneous melanoma, wherein the sample is a tissue sample.

It is also disclosed an *in vitro* method for diagnosing cutaneous melanoma which comprises:
a) determining the expression level of a marker selected from the group consisting of *BCL3, PIR, PEBP1* and combinations thereof in a sample from a subject, and
b) comparing the level obtained in step a) with a reference value for said marker,
wherein a change in the expression level of *BCL3* and/or a decreased expression level of *PIR* and/or *PEBP1* with respect to the reference value for said marker is indicative that said subject suffers from cutaneous melanoma.

As it is used herein, the term "diagnosing" is understood as the method whereby a disease, nosologic entity, pathology or syndrome is identified based on indicators, parameters or symptoms.

As it is used herein, "cutaneous melanoma" refers to a tumor appearing on the skin from cells called melanocytes. These cells originate in the neural crest during embryonic development and migrate to the skin, eyes, central nervous system and to other body parts during fetal life. These tumors appear predominantly in the cutaneous covering, but they can also be present in the mucosae, in the pigmented layers of the eyeball, etc. The torso (chest and back) is the most common site in men. The legs are the most common site in women. The neck and face are other common sites. Melanoma has a radial growth phase corresponding to the early stage of the disease where cells are initially confined in the epidermis; or locally invade the papillary dermis, a microinvasion represented by a clear tumor nodule from the clinical viewpoint. This phase is called a nodular stage. These nodules are not always clinically visible despite sharing histological criteria of vertical growth. According to the World Health Organization, melanoma can be *in situ* melanoma, lentigo maligna melanoma, superficial spreading melanoma, nodular melanoma, fusocellular melanoma, nevoid melanoma, spitzoid melanoma, desmoplastic melanoma, neurotropic melanoma, melanoma with neural differentiation, recurring melanoma, balloon cell melanoma, signet-ring cell melanoma and rhabdoid melanoma.

In a second aspect, the invention relates to an *in vitro* method for determining the probability of metastasis in a subject diagnosed with cutaneous melanoma (hereinafter, "second method") which comprises:
a) determining the expression level of PIR in a sample obtained from a subject, and
b) comparing the level obtained in step a) with a reference value for said marker,
wherein a decreased expression level of PIR with respect to the reference value for said marker is indicative that said subject has a high probability of developing metastasis, wherein the sample is a tissue sample.

It is also disclosed an *in vitro* method for determining the probability of metastasis in a subject diagnosed with cutaneous melanoma which comprises:
a) determining the expression level of a marker selected from the group consisting of *M-MITF, PIR, BCL2* and combinations thereof in a sample obtained from a subject, and
b) comparing the level obtained in step a) with a reference value for said marker,
wherein a decreased expression level of *M-MITF, PIR* and/or *BCL2* with respect to the reference value for said marker is indicative that said subject has a high probability of developing metastasis.

As it is used herein, "probability of developing metastasis" refers to assigning a probability of the tumor cells being able to spread and form metastasis in a subject suffering from cutaneous melanoma. This assignation as is understood by a person skilled in the art does not need to be correct in all the analyzed samples. However, it requires statistically significant amount of the analyzed samples to be correctly classified. The statistically significant amount can be established by a person skilled in the art by means of using different statistical tools, e.g., by means of determining the confidence intervals, determining the p-value, Student's T test, Fisher discriminant functions, etc. The confidence intervals are preferably at least 90%, at least 95%, at least 97%, at least 98% or at least 99%. The p-value is preferably less than 0.1, 0.05, 0.01, 0.005 or 0.0001.

In a third aspect, the invention relates to an *in vitro* method for differentiating benign nevus from cutaneous melanoma in a cutaneous lesion (hereinafter, "third method") which comprises:
a) determining the expression level of PIR and combinations thereof in a tissue sample of said cutaneous lesion, and
b) comparing the level obtained in step a) with a reference value for said marker,
wherein a decreased expression level of PIR with respect to the reference value for said marker is indicative that said cutaneous lesion is a melanoma.

It is also disclosed an *in vitro* method for differentiating benign nevus from melanoma in a cutaneous lesion which comprises:
a) determining the expression level of a marker selected from the group consisting of *BCL3, PIR, PEBP1* and combinations thereof in a tissue sample of said cutaneous lesion, and
b) comparing the level obtained in step a) with a reference value for said marker,
wherein a change in the expression level of *BCL3* and/or a decreased expression level of *PIR* and/or *PEBP1* with respect to the reference value for said marker is indicative that said cutaneous lesion is a melanoma.

As it is used herein, "benign nevus" is understood as the proliferation of different types of cells in the skin.

In a particular embodiment, the benign nevus is a benign amelanotic lesion, a nevus or a premalignant lesion (dysplastic nevus and Spitz nevus).

As it is used herein, "amelanotic lesion" is understood as a skin disorder that lacks melanin pigment.

As it is used herein, "nevi" refers to small pigmented spots on the skin having defined edges and homogeneous light brown or brownish color formed by accumulated nevus cells with melanin. Nevi are cutaneous proliferations of one or more normal cutaneous components which may be present at birth or appear later on. The most common form is melanocytic nevi but there are other types of nevus including epidermal nevus, nevus sebaceous, connective tissue nevus and vascular nevus. The melanocytic nevus -mole- is a lesion which is characterized by the clonal proliferation of melanocytes (nevus cells) affecting different skin structures. There are several varieties of melanocytic nevi according to their characteristics and they include acquired melanocytic nevus, congenital nevus, halo nevus, blue nevus and atypical nevus.

As it is used herein, "dysplastic nevus" refers to atypical, irregular-shaped moles that are larger than normal moles, measuring from 5 to 15 mm, have several colors including pink, brown or black and a flat or raised surface.

As it is used herein, "Spitz nevus" relates to a papular, dome-shaped solitary lesion of less than 1 cm in diameter which is asymptomatic and occurs between the first and second decade of life regardless of sex, being located more frequently in the face and neck, being able to be present in the rest of the body. It has an extremely varied morphology, soft or hard to touch, having various colors and a smooth or rough surface that is homogeneous and well demarcated. Its growth can be slow or very fast.

The methods of the invention [first method of the invention, second method of the invention and third method of the invention] comprise in a first step determining the expression level of *PIR* in a sample from a subject.

As used herein, the term "subject" or "patient" refers to all animals classified as mammals and includes but is not limited to domestic and farm animals, primates and humans, for example, human beings, non-human primates, cows, horses, pigs, sheep, goats, dogs, cats or rodents. The subject is preferably a man or woman of any age or race.

As it is used herein, the term "sample" refers to the biological material isolated from a subject. The biological sample can contain any biological material suitable for determining the expression level of *PIR, PEBP1, BCL3, M-MITF* and/or *BCL2.* The sample of the invention is isolated from any suitable biological tissue.

The sample of the first method of the invention and of the second method of the invention is a tissue sample. Said sample can be obtained by means of conventional methods, for example, biopsy, using methods well known by the persons skilled in related medical techniques or by means of surgical excision. The methods for obtaining a biopsy sample include splitting a tumor into large pieces, or microdissection, or other cell separating methods known in the art. Tumor cells can additionally be obtained by means of cytology through fine needle-aspiration. To simplify sample preservation and handling, samples can be fixed in formalin and embedded in paraffin or be frozen first and then embedded in a medium that can be cryosolidified, such as OCT compound, by means of immersion in a highly cryogenic medium which allows rapid freezing.

According to the third method of the invention, the sample is a tissue of a cutaneous lesion.

As it is used herein, "cutaneous lesion" is understood as a melanotic or amelanotic region that can be a malignant melanoma.

It is also disclosed a method for diagnosing cutaneous melanoma that comprises in a first step determining the expression level of a marker selected from the group consisting of BCL3, PIR, PEBP1 and combinations thereof in a sample from a subject.

As it is used herein, *"BCL3"* refers to the gene encoding the Bcl3 protein acting as a transcriptional coactivator which is activated by means of the association thereof with NF-κB protein homodimers. In humans, the Bcl3 protein corresponds with Uniprot database reference sequence P20749 as of September 19, 2013.

As it is used herein, *"PIR"* refers to the gene encoding the Pirin protein, member of the cupin superfamily, which has been described as a transcription coregulator capable of modulating cell migration and apoptosis. In humans, the Pirin protein corresponds with Uniprot database reference sequence 000625 as of September 19, 2013.

As it is used herein, *"PEBP1"* refers to the gene encoding the phosphatidylethanolamine-binding protein 1, a negative regulator of the MAPK and NF-κB signaling pathways. In humans, the Pebp1 protein corresponds with Uniprot database reference sequence P30086 as of September 19, 2013.

As is understood by the person skilled in the art, the first method comprises determining the expression level of *PIR.* Therefore in a particular embodiment, the first method additionally comprises determining the expression level of *BCL3.* In another particular embodiment, the first method additionally comprises determining the expression level of *PEBP1.*

In another particular embodiment, the first method comprises determining the expression levels of 2 markers from the group consisting of the markers *BCL3, PIR* and *PEBP1,* being *PIR* always one of the markers. Therefore, in another particular embodiment the first method comprises determining the expression level of the markers *BCL3* and *PIR.* In another particular embodiment, the first method comprises determining the expression level of the markers *PIR* and *PEBP1.*

In another particular embodiment, the first method comprises determining the expression level of all 3 markers *BCL3, PIR* and *PEBP1.*

In another particular embodiment, the first method further comprises determining the expression level of an additional marker selected from the group consisting of *M-MITF, BCL2* and combinations thereof.

In a particular embodiment, the *in vitro* method for diagnosing cutaneous melanoma which comprises determining the expression level of PIR further comprises determining the expression level of the markers BCL-3 and/or PEBP1. More preferably, the method further comprises determining the expression level of BCL-3 and PEBP1.

The second method of the invention comprises in a first step determining the expression level of PIR.

It is also disclosed a method for determining the probability of metastasis that comprises in a first step determining the expression level of a marker selected from the group consisting of *M-MITF, PIR, BCL2* and combinations thereof in a sample from a subject.

As it is used herein, *"MITF"* refers to the gene encoding the MITF transcription factor. Said gene gives rise to the transcription of the MITF-A, -B, -C, -D, -E, -H, -J, -Mc and -M isoforms. For the sake of simplicity, in the present invention the transcript of the *MITF* gene that gives rise to the melanocyte-specific *MITF* M isoform (M-mitf) is referred to as *"M-MITF",* i.e., both the mRNA encoding the M-mitf isoform and the cDNA corresponding to said mRNA. Microphthalmia-associated transcription factor (MITF) is a transcription factor with a basic helix-loop-helix leucine zipper domain involved in melanocyte and osteoclast development. The M isoform differs from the other isoforms in that it has an insertion of 6 amino acids before the basic region. In humans, the M isoform corresponds with the Uniprot database reference sequence 075030 as of September 19, 2013.

As it is used herein, *"BCL2"* refers to the gene encoding the Bcl-2 protein which is considered a proto-oncogene regulating mitochondrial permeabilization processes and a key point in the intrinsic cell apoptosis pathway. In humans, it corresponds with Uniprot database reference sequence P10415 as of September 19, 2013.

The term PIR has been defined previously and it is also applicable to the second method of the invention.

As is understood by the person skilled in the art, the second method comprises determining the expression level of *PIR.* Therefore in a particular embodiment, the second method additionally comprises determining the expression level of M-*MITF.* In another particular embodiment, the second method additionally comprises determining the expression level of *BCL2.*

In another particular embodiment, the second method comprises determining the expression level of 2 markers from the group consisting of *M-MITF, PIR* and *BCL2,* being *PIR* always one of the markers. Therefore in a particular embodiment, the second method comprises determining the expression level of the markers *M-MITF* and *PIR.* In another particular embodiment, the second method comprises determining the expression level of the markers *PIR* and *BCL2.*

In another particular embodiment, the second method comprises determining the expression level of all 3 markers M-*MITF, PIR* and *BCL2.*

In another particular embodiment, the second method comprises determining the expression level of an additional marker selected from the group consisting of *BCL2, PEBP1* and combinations thereof.

A particular embodiment of the *in vitro* method for determining the probability of metastasis in a subject diagnosed with cutaneous melanoma which comprises determining the expression level of PIR, further comprises determining the expression level of M-MITF and/or BCL2. More preferably, the *in vitro* method for determining the probability of metastasis in a subject diagnosed with cutaneous melanoma which comprises determining the expression level of PIR, further comprises determining the expression level of M-MITF and BCL2.

The third method of the invention comprises in a first step determining the expression level of PIR.

It is also disclosed a method for differentiating benign nevus from melanoma in a cutaneous lesion that comprises in a first step determining the expression level of a marker selected from the group consisting of *BCL3, PIR, PEBP1* and combinations thereof in a tissue sample of said cutaneous lesion.

As is understood by the person skilled in the art, the third method comprises determining the expression level of *PIR.* Therefore in a particular embodiment, the third method additionally comprises determining the expression level of *BCL3.* In another particular embodiment, the third method additionally comprises determining the expression level of *PEBP1.*

In another particular embodiment, the third method comprises determining the expression level of 2 markers from the group consisting of *BCL3, PIR* and *PEBP1,* being *PIR* always one of the markers. Therefore in a particular embodiment, the third method comprises determining the expression level of the markers *BCL3* and *PIR.* In another particular embodiment, the third method comprises determining the expression level of the markers *PIR* and *PEBP1.*

In another particular embodiment, the third method comprises determining the expression level of all 3 markers *BCL3, PIR* and *PEBP1.*

In another particular embodiment, the third method comprises determining the expression level of an additional marker selected from the group consisting of *M-MITF, BCL2* and combinations thereof.

A particular embodiment of the *in vitro* method for differentiating benign nevus from cutaneous melanoma in a cutaneous lesion which comprises determining the expression level of PIR, further comprises determining the expression level of *BCL3* and/or *PEBP1.* More preferably, the *in vitro* method for differentiating benign nevus from cutaneous melanoma in a cutaneous lesion which comprises determining the expression level of PIR, further comprises determining the expression level of *BCL3* and *PEBP1.*

As it is used herein, the term "expression level" of a marker refers to the measurable amount of gene product in a sample from the subject, in which the transcription product or a translation product of said gene. Accordingly, said expression level can correspond to the expression level of a nucleic acid (such as mRNA or cDNA) of the corresponding gene or to the expression level of a protein or polypeptide encoded by said gene. The expression level is derived from the sample from a subject and/or reference sample or samples, and can be detected *de novo,* or correspond to a previous determination.

As is understood by the person skilled in the art, the expression levels of *PIR, PEPBP1, BCL3, M-MITF* and *BCL2* can be determined by measuring the levels of the mRNAs (or the corresponding cDNAs) of the corresponding genes or by measuring the levels of the proteins encoded by said genes, and the levels of the variants thereof.

By way of non-limiting illustration, in a particular embodiment the expression level of the markers is determined by quantifying the level of the mRNAs encoded by the genes in question. The level of mRNA of a gene can be quantified by means of using conventional methods, for example, methods comprising mRNA amplification and the quantification of said mRNA amplification product, such as electrophoresis and staining, or alternatively, by means of Northern blot and using specific probes of the mRNA of the genes of interest or the corresponding cDNA/cRNA, mapping with S1 nuclease, RT-PCR, hybridization, microarrays, etc. Similarly, the levels of the cDNA/cRNA corresponding to said mRNA encoded by the markers can also be quantified by means of using conventional techniques; in this case, the method of the invention includes a step of synthesizing the corresponding cDNA by means of reverse transcription (RT) of the corresponding mRNA followed by synthesizing (RNA polymerase) and amplifying the cRNA complementary to said cDNA. Conventional methods for quantifying expression levels can be found, for example, in Sambrook et al., 2001 "Molecular cloning: A Laboratory Manual", 3rd ed., Cold Spring Harbor Laboratory Press, N.Y., Vol. 1-3.

To normalize the expression values of mRNA between the different samples, it is possible to compare the expression levels of the mRNA of interest in the test samples with the expression of a control RNA. As it is used herein, a "control RNA" refers to an RNA the expression levels of which do not change or change only in limited amounts. The control RNA is preferably an mRNA derived from housekeeping genes and encodes proteins which are expressed constitutively and carry out essential cell functions. The preferred housekeeping genes for use in the present invention include the 18S ribosomal protein, β-2-microglobulin, ubiquitin, cyclophilin, GAPDH, PSMB4, tubulin and β-actin.

In a particular embodiment of the methods of the invention [first method of the invention, second method of the invention and third method of the invention], the expression levels of the markers are determined by means of a quantitative polymerase chain reaction (PCR) or a DNA or RNA array.

Alternatively, it is also possible to determine the expression levels of the markers by means of determining the expression levels of the proteins encoded by the genes, since if the expression of the genes increases, the amount of the corresponding protein is expected to increase, and if the expression of the genes decreases, the amount of the corresponding protein is expected to decrease.

By way of non-limiting illustration, the levels of said proteins can be quantified, for example, by means of using antibodies capable of binding to said proteins (or to fragments thereof containing an antigenic determinant) and the subsequent quantification of the complexes formed. The antibodies which are used in these assays may or may not be labeled. Illustrative examples of markers which can be used include radioactive isotopes, enzymes, fluorophores, chemiluminescence reagents, enzyme substrates or cofactors, enzyme inhibitors, particles, dyes, etc. There is a wide range of known assays which can be used in the present invention using unlabeled antibodies (primary antibody) and labeled antibodies (secondary antibody); these techniques include Western-blot transfer, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemistry techniques, techniques based on the use of protein microarrays or biochips including specific antibodies or assays based on colloidal precipitation in formats such as dipsticks. Other ways for detecting and quantifying the proteins include affinity chromatography techniques, ligand binding assays, etc.

When an immunological method is used, any antibody or reagent that is known to bind to the target proteins with a high affinity can be used for detecting the amount the target proteins. However, the use of an antibody is preferred, for example, polyclonal sera, supernatants of hybridomas or monoclonal antibodies, antibody fragments, Fv, Fab, Fab' and F(ab')2, scFv, diabodies, triabodies, tetrabodies and humanized antibodies.

In a preferred embodiment of the methods of the invention, the levels of the markers are determined by an immunological technique. In a more particular embodiment, the expression level is determined by immunohistochemistry, Western blot, ELISA or a protein array. In a more preferred embodiment, the immunological technique is immunohistochemistry or IHC. Generally, IHC is a known histopathological method based on the use of a specific antibody, usually labeled with a marker (for example, an enzyme), which can transform a substrate into a visible compound without affecting the antibody's capacity to form a complex with the antigen applied to an organic tissue sample. With the use of one of the specific techniques (peroxidase, antiperoxidase, fluorescein, etc.), the antigen-antibody complex formed can be located and identified in the tissue or cell samples to be studied, whereby the antigenic markers characteristics of different cells are identified and the type of cell found in the sample can be determined.

To detect the presence of the markers of the invention by means of IHC (or immunohistochemistry analysis), the sample to be analyzed can be a fresh sample, a frozen sample or a sample embedded in paraffin and fixed using a formalin-type protecting agent or the like. In a particular embodiment, the immunohistochemistry analysis is carried out by staining the sample to be analyzed with one or more antibodies specific for one or more markers of the invention and determining the frequency of stained cells and the staining intensity. Advantageously, detection of the presence of the markers in the sample to be analyzed by means of IHC is carried out in parallel with tissue or cell samples serving as positive marker and as negative marker, and if desired, healthy tissues of the same origin as the tumor which is being analyzed can be used as reference. A background control is also frequently used. Typically, the sample is assigned with a value indicative of the total expression calculated depending on the frequency of stained cells and on the intensity in each of the stained cells. The typical criteria for assigning expression values to the samples have been described, for example, in Handbook of Immunohistochemistry and In Situ Hybridization in Human Carcinomas, M. Hayat Ed., 2004, Academic Press.

The determination of the expression level of one or more markers of the invention by means of IHC has advantages since it does not alter the clinical routine of tissue extraction and sample processing in pathological anatomy laboratories.

In those cases in which a large number of samples is to be analyzed (for example, when several samples from one and the same patient or samples from different patients are to be analyzed), the use of array formats and/or automated methods is possible. In one embodiment, the use of tissue microarrays (TMA) which can be obtained using different techniques is possible. The samples forming part of the microarrays can be analyzed in a different manner including immunohistochemistry, *in situ* hybridization, *in situ* PCR, RNA or DNA analysis, morphological inspection and combinations of any of the foregoing techniques. Methods for processing tissue microarrays have been described, for example, in Konenen, J. et al., (Nat. Med. 1987, 4:844-7). The tissue microarrays are prepared from cylindrical cores of 0.6 to 2 mm in diameter from tissue samples embedded in paraffin and re-embedded in a single receiver block. The tissue originating from several samples can thus be inserted in a single paraffin block.

As mentioned above, the expression levels of *PIR, PEBP1, BCL3, M-MITF* and *BCL2* can be determined by measuring both the levels of protein and the levels of variants thereof, such as fragments, analogs and/or derivatives.

As it is used herein, a variant of a marker (protein) can be (i) a polypeptide or protein (generally, "peptide") in which one or more amino acid residues are substituted with a conserved or non-conserved amino acid residue, preferably a conserved amino acid residue, and such substituted amino acid residue may or may not be one encoded by the genetic code, (ii) a peptide in which there is one or more modified amino acid residues, for example, residues which are modified by the binding of substituent groups, (iii) a peptide in which the protein is an alternative splicing variant of the proteins identified as markers in the present invention, and/or (iv) a fragment of said proteins. The fragments include proteins generated through proteolytic cleavage (including proteolysis at multiple sites) of an original sequence. Said variants can be identified by the persons skilled in the art from the teaching of the present patent application.

As is known in the art, the "identity" between two proteins is determined by comparing the amino acid sequence of a first protein with the amino acid sequence of a second protein. According to the present invention, a "variant" is a peptide (polypeptide or protein) having an amino acid sequence which is at least 60%, 65%, 70%, 75%, 80%, 85%, 90%, 95% or even more, identical to the amino acid sequence of the proteins identified as markers by this invention. The degree of identity between two proteins can be determined using computer algorithms and methods known by the persons skilled in the art. By way of illustration, in a particular embodiment the degree of identity between 2 amino acid sequences is determined by means of using the BLASTP algorithm [BLASTManual, Altschul, S., et al., NCBI NLM NIH Bethesda, Md. 20894, Altschul, S., et al., J. Mol. Biol. 215: 403-410 (1990)].

The proteins can be modified after translation; such post-translational modifications fall within the scope of the present invention and include, by way of illustration, signal peptide cleavage, glycosylation, acetylation, isoprenylation, proteolysis, myristoylation, protein folding and proteolytic processing, etc. The proteins can also include non-natural amino acids formed by post-translational modifications or by introducing non-natural amino acids during translation.

Alternatively, the expression level of the markers of the invention can be determined by means of analyzing the activity of the proteins or of a functionally equivalent variant thereof.

By way of non-limiting illustrative example, the activity of Bcl-3 can be determined by means of inhibiting the expression of IL-10 as described in Riemann M. et al., J Immunol. Sep 15, 2005;175(6):3560-8, or by means of suppressing the activation of p53 as described in Kashatus D. et al., Genes Dev. Jan 15, 2006; 20(2):225-35. Epub Dec 29, 2005.

By way of non-limiting illustrative example, the activity of PEBP1 can be determined by means of inhibiting the serine protease activity, as described in Hengst U. et al., J Biol Chem. Jan 5, 2001; 276(1):535-40.

By way of non-limiting illustrative example, the activity of Bcl-2 can be determined by means of inhibiting the transcriptional activity of p53 as described in Barbara A. et al., Journal of Biological Chemistry, 274, 6469-6475.

By way of non-limiting illustrative example, the activity of M-mitf can be determined by means of expressing Dial as described in Carreira S. et al., Genes Dev. Dec 15, 2006; 20(24):3426-39.

By way of non-limiting illustrative example, the activity of Pirin can be determined by means of quercetinase enzymatic activity as described in Adams M. et al., J Biol Chem 2005, 280 (31) :28675-28682.

The second step of the methods of the invention [first method of the invention, second method of the invention and third method of the invention] comprises comparing the expression level with a reference value for said marker.

As it is used herein, the term "reference value" refers to predetermined criteria used as a reference to evaluate the values or data obtained from samples collected from a subject. The reference value or reference level can be an absolute value, a relative value, a value having an upper or lower limit, a range of values, a mean value, a median value, an average value, or a value as compared to a specific control or baseline value. A reference value can be based on an individual sample value, such as, for example, a value obtained from a sample from the subject being analyzed, but at an earlier point in time. The reference value can be based on a large number of samples, such as values from a population of subjects from the same age group, or based on a pool of samples including or excluding the sample to be analyzed. In a particular embodiment, the reference value for a marker is the expression level of said marker in a sample from a control subject or population of control subjects, i.e., those who do not suffer from melanoma. Generally, the typical reference samples will be obtained from subjects who are clinically well-documented.

As it is used herein, "change" refers to a variation in the expression levels of a marker with respect to a reference value. Said variation can be an increased or a decreased expression level.

According to the present invention, the level of a marker increases when the level of said marker in a sample is greater than a reference value. The levels of a marker are considered greater than the reference value thereof when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more, greater than the reference value.

Likewise, in the context of the present invention the level of a marker decreases when the level of said marker in a sample is less than a reference value. The levels of a marker are considered less than the reference value thereof when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more, greater than the reference value.

According to the first method, a decreased expression level of *PIR* with respect to the reference value for said marker is indicative that said subject suffers from cutaneous melanoma. An increase in the expression level of *BCL3,* and/or a decreased expression level of *PEBP1,* with respect to the reference value for said marker/markers, is indicative that said subject suffers from cutaneous melanoma.

In a more particular embodiment of the first method, an increased expression level of *BCL3* of at least 75% with respect to the reference value for said marker and decreased expression levels of *PIR* and of *PEBP1* of at least 75% with respect to the reference value for each of said markers, is indicative that said subject suffers from cutaneous melanoma.

According to the second method of the invention, a decreased expression level of *PIR* with respect to the reference values for each marker is indicative that said subject has a high probability of developing metastasis. A decreased expression level of *M-MITF* or *BCL2* with respect to the reference values for each marker is indicative that said subject has a high probability of developing metastasis.

In a particular embodiment of the second method of the invention in which the expression levels of the markers *PIR, M-MITF* and *BCL2* are determined, a decreased expression level of the marker *PIR* of at least 75% with respect to the reference value for said marker and decreased expression levels of the markers *M-MITF* and *BCL2* of at least 90%, more preferably more than 90%, with respect to the reference value for each of said markers, is indicative that said subject has a high probability of developing metastasis.

According to the third method of the invention, a decreased expression level of PIR with respect to the reference value for said marker is indicative that said cutaneous lesion is cutaneous melanoma. An increase in the expression level of BCL3 or a decreased expression level of *PEBP1* with respect to the reference value for said markers is indicative that said cutaneous lesion is cutaneous melanoma.

### Kit of the invention

It is disclosed a kit comprising a reagent capable of determining the expression level of a marker selected from the group consisting of *BCL3, PIR, PEBP1, M-MITF, BCL2* and combinations thereof.

In one aspect, the invention relates to a kit comprising a reagent capable of determining the expression level of PIR further comprisinga reagent capable of determining the expression level of a marker selected from the group consisting of *BCL3, PEBP1, M-MITF, BCL2* and combinations thereof, wherein the reagent capable of determining the expression level is an antibody or a nucleic acid.

In an embodiment, the kit comprising a reagent capable of determining the expression level of PIR additionally comprises the reagents for determining the expression levels of BCL3 and PEBP1; BCL3 and M-MITF; BCL3 and BCL2; PEBP1 and M-MITF ; PEBP1 and BCL2 or M-MITF and BCL2.

In another embodiment, the kit comprising a reagent capable of determining the expression level of PIR additionally comprises the reagents for determining the expression levels of BCL3, PEBP1 and M-MITF; BCL3, PEBP1 and BCL2; BCL3, M-MITF and BCL2; PEBP1, M-MITF and BCL2.

In another embodiment, the kit comprising a reagent capable of determining the expression level of PIR additionally comprises the reagents for determining the expression levels of *BCL3, PEBP1, M-MITF* and *BCL2.*

As it is used herein, the term "kit" refers to a product containing the different reagents necessary for carrying out the methods of the invention packaged such as to allow transport and storage. The materials suitable for packaging the components of the kit include glass, plastic (polyethylene, polypropylene, polycarbonate and the like), bottles, vials, paper, sachets and the like. Additionally, the kits of the invention can contain instructions for the simultaneous, sequential or separate use of the different components found in the kit. Said instructions can be found in the form of a printed material or in the form of an electronic support capable of storing instructions such that they can be read by a subject, such as electronic storage means (magnetic discs, tapes and the like), optical means (CD-ROM, DVD) and the like. Additionally or alternatively, the means can contain Internet addresses that provide said instructions.

Kits comprising a single reagent will generally have the reagent enclosed in a receptacle (for example, a flask, an ampoule, or other suitable storage container), although kits including the reagent bound to a substrate (for example, an inner surface of a test reaction container) are also contemplated. Likewise, kits including more than one reagent can also have the reagents in receptacles (separately or in a mixture) or can have the reagents bound to a substrate.

As it is used herein, "reagent capable of determining the expression level" is understood as a compound capable of detecting the gene product of a gene or the protein encoded by said gene.

In some embodiments, the reagents suitable for determining the expression levels of one or more markers of the invention comprise at least 50%, at least 55%, at least 60%, at least 65%, at least 70%, at least 75%, at least 80%, at least 85%, at least 90%, at least 95%, at least 96%, at least 97%, at least 98% or at least 99%, of the total amount of the reagents forming the kit.

In a particular embodiment, said kit comprises the reagents necessary to perform an immunoassay, preferably an immunohistochemistry analysis, to detect the presence of Pirin, PEBP1, Bcl-3, M-mitf and/or Bcl-2; to that end, said kit will include antibodies recognizing said markers.

Therefore in a preferred embodiment of the kit of the invention, the "reagent capable of determining the expression level" is an antibody that binds to a marker of the invention.

As it is used herein, the term "antibody" can be a natural polyclonal or monoclonal antibody or a non-natural antibody, for example, a single-domain antibody, a single-chain variable fragment antibody, a microantibody, etc. Methods for producing such antibodies are well known in the art.

In some embodiments, the antibodies specific for the markers of the invention are labeled with a detectable marker (for example, a fluorescent dye or a detectable enzyme), or are modified to make detection easier (for example, with biotin to allow detection with an avidin or a streptavidin). In other embodiments, the reagent will not be labeled or modified directly.

In certain embodiments, the kits include the reagents in the form of an array. The array includes at least two different reagents suitable for determining the expression levels of one or more proteins (each reagent being specific for a different protein) bound to a substrate in a predetermined pattern (for example, a lattice). Accordingly, the present invention provides arrays comprising the reagents suitable for determining the expression levels of one or more markers of the invention

The location of the different reagents (the "capture reagents") allows measuring the levels of a number of different markers in the same reaction. Kits including the reagents in the form of an array are commonly in a sandwich format, so such kits can also contain detection reagents. The kit usually includes different detection reagents, each detection reagent being specific for a different antibody. The detection reagents in such embodiments are usually reagents specific for the same proteins as the reagents bound to the substrate (although detection reagents typically bind to a different portion or in the site in the protein of the reagents cross-linked to the substrate), and are generally affinity detection reagents. Like the detection reagents of any other assay format, the detection reagents can be modified with a detectable moiety, modified to allow the separate binding of a detectable moiety, or not modified. The array-type kits including detection reagents which are modified or not modified to allow the binding of a detectable moiety can also contain additional detectable moieties (for example, detectable moieties binding to the detection reagent, such as labeled antibodies binding to non-modified detection reagents or streptavidin modified with a detectable moiety for the detection of biotin-modified detection reagents).

In another preferred embodiment of the kit of the invention, the "reagent capable of determining the expression level" comprises a nucleic acid.

As it is used herein, the term "nucleic acid" refers to a probe, a primer, capable of hybridizing specifically with the polynucleotide encoding a marker of the invention.

In a particular embodiment, the kit of the invention comprises a DNA or RNA array. In other words, the nucleic acids, oligonucleotides, forming the kit of the invention are coupled to an array. The microarrays comprise a plurality of spatially distributed nucleic acids stably associated with a support (for example, a biochip). The nucleic acids have a sequence complementary to particular subsequences of the markers the expression of which is to be detected, so they are capable of hybridizing with said nucleic acids. In the methods of the invention, a microarray comprising a nucleic acid array is contacted with a nucleic acid preparation isolated from the subject object of the study. The microarray is incubated with the nucleic acid preparation under conditions suitable for hybridization. Then, after removing the nucleic acids that were not retained on the support, the hybridization pattern is detected providing information about the genetic profile of the analyzed sample.

The invention contemplates a variety of arrays both as regards the type of probes and as regards the type of support used. The probes included in the arrays which are capable of hybridizing with the nucleic acids can be nucleic acids or analogs thereof that maintain the hybridization capacity, such as, for example, nucleic acids in which the phosphodiester bond has been substituted with a phosphorothioate bond, methylimino bond, methylphosphonate bond, phosphoramidate bond, guanidine bond and the like, nucleic acids in which the ribose of the nucleotides has been substituted with another hexose, peptide nucleic acids (PNAs). The probes can be 7, 10, 15, 20, 25, 30, 35, 40, 45, 50, 55, 60, 65, 70, 75, 100 nucleotides long and can vary in the range of 10 to 1000 nucleotides, preferably in the range of 15 to 150 nucleotides, more preferably in the range of 15 to 100 nucleotides and can be single- or double-stranded nucleic acids.

The specific probes for the different target genes are selected such that they bind specifically to the target nucleic acid with minimum hybridization to unrelated genes. However, there are probes 20 nucleotides long that are not unique for a specific mRNA. Therefore, probes targeting said sequences will show cross-hybridization with identical sequences found in the mRNA of unrelated genes. On the other hand, there are probes that do not hybridize specifically with the target genes under the conditions of use (due to the secondary structures or interactions with the substrate of the array). Such probes must not be included in the array. Therefore, the person skilled in the art will find that the probes to be incorporated in a specific array must be optimized before incorporation into the array. Generally, the optimization of the probes is carried out by generating an array containing a plurality of probes targeting the different regions of a specific target polynucleotide. Firstly, this array is contacted with a sample containing the target nucleic acid in an isolated manner and, secondly, with a complex nucleic acid mixture. Probes showing a highly specific hybridization with the target nucleic acid, and not low or zero hybridization with the complex sample, are selected for incorporation into the arrays of the invention. Additionally, it is possible to include in the array hybridization controls for each of the probes to be studied. In a preferred embodiment, the hybridization controls contain an altered position in the central region of the probe. If high levels of hybridization between the probe under study and its hybridization control are observed, the probe is not included in the array.

In a particular embodiment, the microarrays which can be used in putting the present invention into practice contain a series of control probes; by way of illustration, said control probes can be of three types: normalization controls, expression level controls and hybridization controls.

Normalization controls are oligonucleotides that are perfectly complementary to labeled reference sequences which are added to the nucleic acid preparation to be analyzed. The signals derived from the normalization controls after the hybridization provide an indication of the variations in the hybridization conditions, marker intensity, detection efficiency and another series of factors that can result in a variation of the hybridization signal between different microarrays. The signals detected from the rest of the probes of the array are preferably divided by the signal emitted by the control probes, thus normalizing the measurements. Virtually any probe can be used as normalization control. However, it is known that hybridization efficiency varies according to the composition of nucleotides and the length of the probe. Therefore, preferred normalization probes are those which represent the mean length of the probes present in the array, although they can be selected such that they include a range of lengths that reflect the rest of the probes present in the array. The normalization probes can be designed such that they reflect the mean composition of nucleotides of the rest of the probes present in the array. A limited number of selected normalization probes is preferably used such that they hybridize suitably, i.e., they do not have a secondary structure and do not show sequence similarity with any of the probes of the array. The normalization probes can be located in any position in the array or in multiple positions in the array to efficiently control variations in hybridization efficiency related to the structure of the array. The normalization controls are preferably located in the corners of the array and/or in the center thereof.

The expression level controls are probes which hybridize specifically with genes which are expressed constitutively in the sample being analyzed. The expression level controls are designed to control the physiological state and the metabolic activity of the cell. The examination of the covariance of the expression level control with the expression level of the target nucleic acid indicates if the variations in the expression levels are due to changes in the expression levels or are due to changes in the overall transcriptional rate in the cell or in its general metabolic activity. Therefore, in the case of cells which have deficiencies in a certain metabolite essential for cell viability, a decrease both in the expression levels of the target gene and in the expression levels of the control is expected to be observed. On the other hand, if an increase in the expression of the expression of the target gene and of the control gene is observed, it is probably due to an increase of the metabolic activity of the cell and not to a differential increase in the expression of the target gene. Any probe corresponding to a gene expressed constitutively, such as genes encoding proteins exerting essential cell functions such as β-2-microglobulin, ubiquitin, 18S ribosomal protein, cyclophilin A, transferrin receptor, actin, GAPDH and the like, can be used. In a preferred embodiment, the expression level controls are *GAPDH,* tyrosine 3-monooxygenase/tryptophan 5-monooxygenase activation protein *(YWHAZ),* ubiquitin, beta-actin and β-2- microglobulin.

Hybridization controls can be included both for the probes specific for the target genes and for the probes specific for the expression level or the normalization controls. Error controls are oligonucleotide probes identical to the probes specific for target genes but which contain mutations in one or several nucleotides, i.e., which contain nucleotides in certain positions which do not hybridize with the corresponding nucleotide in the target gene. The hybridization controls are selected such that, by applying the suitable hybridization conditions, the target gene should hybridize with the specific probe but not hybridize or hybridize with a reduced efficiency with the hybridization control. The hybridization controls preferably contain one or several modified positions in the center of the probe. The hybridization controls therefore provide an indication of the degree of unspecific hybridization or of cross-hybridization to a nucleic acid in the sample to a probe different from that containing the exactly complementary sequence.

Materials suitable for inclusion in an exemplary kit according to the present invention comprise one or more of the following: specific primer pairs for PCR (oligonucleotides) that hybridize with the domains of the DNA or cDNA sequence flanking the genetic polymorphisms of interest; reagents capable of amplifying a domain of a specific sequence, either in the genomic DNA or cDNA without needing to perform PCR; reagents necessary for distinguishing the different alleles in the sequence of the domains amplified and not amplified by PCR (for example, restriction endonucleases, oligonucleotides preferably hybridizing to an allele of the polymorphism, including those modified to contain enzymes or fluorescent chemical groups amplifying the signal of the oligonucleotides and making the discrimination of the alleles more robust); reagents necessary for physically separating the products derived from the different alleles (for example, agarose or polyacrylamide and a buffer to be used in electrophoresis, HPLC columns, SSCP gels, formamide gels or a support of the MALDI-TOF array), or even, reagents capable of identifying polypeptides and the variants thereof according to the invention, for example, antibodies (including monoclonal antibodies, polyclonal antibodies or whole antibodies and fragments thereof, as well as recombinant antibodies, etc.) capable of recognizing and binding to said polypeptides or variants thereof.

### Uses of the kits of the invention

In another aspect, the invention relates to the use of a kit comprising an antibody or a nucleic acid capable of determining the expression level of *PIR* or a kit of the invention for diagnosing cutaneous melanoma, for determining the probability of metastasis in a subject diagnosed with cutaneous melanoma or for differentiating a benign nevus from cutaneous melanoma.

The invention is now described in detail by means of the following examples which must be considered as merely illustrative and non-limiting with respect to the scope of the invention.

The materials and methods described below were used to carry out the examples.

### Materials and Methods

### Cell lines

The malignant melanoma cell lines were:
- 1205Lu (ATCC CRL-2812): melanoma cell line established by M. Herlyn. It is derived from a lung metastasis after subcutaneous injection of commercial human melanoma cell line WM793B in an immunodeficient mouse. The 1205Lu cells are highly invasive and show spontaneous metastasis in lung and liver. This line was acquired in 2007 through the American Type Culture Collection (ATCC).
- A375 (ATCC CRL-1619): melanoma cell line established by D.J. Giard in 1973 from a solid tumor found in a 54-year-old woman. This line has a hyperploid karyotype with a modal number of 62 chromosomes and is capable of producing amelanocytic subcutaneous tumors when inoculated in athymic mice. This line was acquired in 1996 through the ATCC.
- COLO-800 (ACC 193): melanoma cell line isolated by G. E. Moore in 1990 from a subcutaneous nodule of a tumor in a 14-year-old boy. This line was acquired in 2010 through the company, Innoprot (Biscay).
- Hs294T (ATCC HTB-140): melanoma cell line established by A. Creasey in 1969 from a lymph node metastatic melanoma in a 56-year-old caucasian woman. It is a hyperploid cell line with a modal number of 65 chromosomes in at least 44% of cells. This line was acquired in 1996 through the ATCC.
- HT-144 (ATCC HTB 63): melanoma cell line established by J. Fogh from a malignant metastatic melanoma in a 26-year-old man. Cytogenetic analyses show polyploidy with abnormalities including acrocentric fragments and secondary constrictions. This line was acquired in 1996 through the ATCC.
- MEL-HO (ACC62): melanoma cell line established by H. W. L. Ziegler-Heitbrock in 1976 from a primary melanoma in a woman of unknown age. This line was acquired in 2010 through the company, Innoprot (Biscay).
- MEL-Juso (ACC74): melanoma cell line established by H. W. L. Ziegler-Heitbrock in 1977 from a primary melanoma in a 58-year-old woman. This line was acquired in 2010 through the company, Innoprot (Biscay).
- RPMI7951 (ACC76): melanoma cell line established by H. Kirchner in 1971 from a lymph node metastasis in an 18-year-old caucasian girl. This line was acquired in 2010 through the company, Innoprot (Biscay).
- WM793B (ATCC CRL-2806): melanoma cell line established by M. Herlyn in 1983 from a primary melanoma in vertical growth located at the sternum of a 37-year-old man. It shows a translocation between chromosomes 1 and 19, in addition to having an extra copy of chromosome 7. This line was acquired in 2007 through the ATCC.
- M980513: melanoma cell line established by Dr. Hoek's group in 1998 (University Hospital Zurich, Switzerland). This line was obtained from an iliac lymph node metastasis found in a 42-year-old woman.
- M080423: melanoma cell line established by Dr. Hoek's group in 2008 (University Hospital Zurich, Switzerland). This line was obtained from a hepatoduodenal lymph node metastasis found in a 65-year-old woman.
- M050829: melanoma cell line established by Dr. Hoek's group in 2005 (University Hospital Zurich, Switzerland). This line was obtained from a retroauricular cutaneous metastasis found in a 39-year-old man.
- M000921: melanoma cell line established by Dr. Hoek's group in 2000 (University Hospital Zurich, Switzerland). This line was obtained from an epigastric metastasis found in a 52-year-old woman.
- M010817: melanoma cell line established by Dr. Hoek's group in 2001 (University Hospital Zurich, Switzerland). This line was obtained from a cutaneous metastasis located in the upper left extremity of a 37-year-old woman.
- M080201: melanoma cell line established by Dr. Hoek's group in 2008 (University Hospital Zurich, Switzerland). This line was obtained from a primary melanoma found in a 64-year-old woman.
- M080310: melanoma cell line established by Dr. Hoek's group in 2008 (University Hospital Zurich, Switzerland). This line was obtained from a primary melanoma found in a 71-year-old woman.
- M080214: melanoma cell line established by Dr. Hoek's group in 2008 (University Hospital Zurich, Switzerland). This line was obtained from a subcutaneous metastasis located in the left thigh of a 55-year-old woman.
- M080307: melanoma cell line established by Dr. Hoek in 2008 (University Hospital Zurich, Switzerland). This line was obtained from an axillary metastasis found in a 55-year-old man.
- JSG: melanoma line obtained in the laboratory of the inventors in 1997 (Dr. Boyano, University of the Basque Country, Biscay). This line was established from a primary tumor located on the foot of a 71-year-old man. The tumor was diagnosed as a stage IIB tumor according to the AJCC classification. The progression of the disease was not good, node metastasis occurring 5 months after the removal of the primary tumor. Intransit metastasis and distant metastasis were detected three months later, and the patient passed away after 5 months.

The skin melanocyte cell lines are the following:
- Most of the human epidermal melanocyte lines used in this project were acquired through Cascade Biologics in 2007.
- HEMn-LP (C-002-5C): melanocytes isolated from the skin of a newborn with low pigmentation.
- HEMn-MP (C-102-5C): melanocytes isolated from the skin of a newborn with medium pigmentation.
- HEMn-DP (C-202-5C): melanocytes isolated the skin of a newborn with high pigmentation.
- The last melanocyte line HEM (P10853) was acquired in 2010 through the company, Innoprot (Biscay). Like HEMn-LP, it is a primary culture of human melanocytes isolated from the skin of a newborn with low pigmentation.

The primary human melanocytes were acquired from Invitrogen (Cat No. C-002-5C from the foreskin of a lightly pigmented newborn, HEMn-LP; Cat. No. C-102-5C from the foreskin of a moderately pigmented newborn, HEMn-MP; and Cat No. C-202-5C from the foreskin of a heavily pigmented newborn, HEMn-DP) and from Innoprot (Cat. No. P10853 from the foreskin of a lightly pigmented newborn).

All the primary human melanocytes were grown in Cascade Media 254 (Invitrogen, Carlsbad, CA, USA) supplemented with Cascade human melanocyte growth supplement (Invitrogen, Carlsbad, CA, USA), in the absence of antibiotics. The A375 (ATCC CRL-1619 ), Hs294T (ATCC HTB-140), JSG (isolated by Boyano MD), RPMI7951 (ACC76) melanoma cell lines were cultured in Dulbecco's modified Eagle's medium (DMEM) supplemented with 10% fetal bovine serum (FBS), 2 mM L-glutamine, 50 mg/ml penicillin and 100 mg/ml streptomycin. The HT-144 (ATCC HTB-63) melanoma cell line was kept in McCoy's 5A medium supplemented with 10% FBS, 0.22 g/L L-glutamine and sodium bicarbonate. The WM793B (ATCC CRL-2806) and 1205Lu (ATCC CRL-2812) melanoma cell lines were cultured in 2% of the median tumor containing a 4:1 mixture of MCDB 153 medium with 1.5 g/L sodium bicarbonate and Leibovitz's L-15 medium with 2 mM L-glutamine, and then supplemented with 2% FBS, 0.005 mg/ml bovine insulin and 1.68 mM CaCl₂. The COLO-800 (ACC193), MEL-HO (ACC62) and MEL-Juso (ACC74) melanoma cell lines were cultured in RPMI 1640 medium supplemented with 10% FBS, 5% glutamine and 1 mM sodium pyruvate. The A375, Hs294T, HT-144, WM793B and 1205Lu melanoma cell lines were acquired from the ATCC (Rockville, MD, USA); RPMI7951, COLO-800, MEL-HO (ACC62) and MEL-Juso (ACC74) were obtained from Innoprot (Derio, Biscay, Spain). All the primary human melanocytes and melanoma cell lines were cultured to 95% confluence at 37°C with 5% CO₂ and 95% humidity.

Table 1 shows the different culture conditions for the melanoma cell lines and melanocytes.

**Table 1**

| Cell line | Culture conditions |
|---|---|
| A375, Hs294T, JSG, RPMI7951 | DMEM medium |
| | 10% FCS |
| | 2 mM L-glutamine |
| | 100 IU/ml penicillin |
| | 100 µg/ml streptomycin |
| HT-144 | McCoy's 5A medium |
| | Sodium bicarbonate |
| | 0.22 g/L L-glutamine |
| | 10% FCS |
| 1205Lu, WM793B | Medium dilution of 4:1 |
| | MCDB 153 with 1.5 g/L sodium bicarbonate |
| | Leibovitz's L-15 medium with 2 mM L-glutamine |
| | plus 0.005 mg/ml bovine insulin |
| | 1.68 mM CaCl₂ |
| | 2% FCS |
| COLO-800, MEL-HO, MEL-JUSO | RPMI 1640 medium with GlutaMax |
| | 25 mM HEPES |
| | 10% FCS |
| M980513, M080423, M050829, M000921, M010817, M080201, M080310, M080214, M080307 | RPMI 1640 medium |
| | 1 mM sodium pyruvate |
| | 10% FCS |
| | 5 mM Glutamine |
| HEMn-LP, HEMn-MP, HEMn-DP, | 254 medium |
| | 1% human melanocyte growth |
| | supplement |

### Biopsies

The results were assessed in the biopsies of melanoma and nevus from paraffin blocks stored in the Pathological Anatomy Department of the Cruces University Hospital. Twenty-five samples were analyzed in total of which 10 were intradermal nevi, 5 dysplastic nevi and 10 malignant melanomas.

Information was provided to the patients and informed consent was obtained both for inclusion in the study and for obtaining biological samples following the rules and regulations. The study was conducted according to Organic Law 15/1999 on Personal Data Protection, particularly as regards sending data to a third party and handling same.

### Gene expression by quantitative RT-PCR

The total RNA was isolated using the RNeasy Mini Kit (Qiagen Inc., Hilden, Germany) and for each sample, the cDNA was synthesized from 1 mg of total RNA using the cDNA synthesis kit, *iScript™* (Bio-Rad Laboratories, Hercules, CA, USA) according to the manufacturer's instructions. The real time RT-PCR assays were carried out using an iCycler (Bio-Rad Laboratories, Hercules, CA, USA) PCR platform. The reaction mixture contained 0.1 µl cDNA from the reverse transcription reaction, together with specific forward and reverse primers and *IQTM SYBR® Green Supermix* (Bio-Rad Laboratories, Hercules, CA, USA) in a final reaction volume of 20 µl. The PCR reaction was initiated with heating at 95°C for 10 minutes, followed by 45 denaturation cycles at 95°C for 30 seconds, at the corresponding temperature for each gene (56-61°C) for 20 seconds and elongation at 72°C for 30 seconds. Each assay included a negative control consisting of the absence of cDNA. The expression data was generated from 2 amplification reactions with the samples and the controls performed in triplicate. The optical data obtained through real time PCR was analyzed using *MyiQ Single-Color from Real-Time PCR System Software Detection, Version 1.0* (Bio-Rad Laboratories, Hercules, CA, USA). The melting curve of each PCR assay was determined and randomly selected samples were analyzed in a 1.5% agarose gel electrophoresis to check the specificity of the amplification products. The expression of three constitutive genes (*ACTB, GAPDH,* and *RPS15*) was also analyzed for normalizing the expression data using *Gene Expression Macro Software Version 1*.*1* (Bio-Rad Laboratories, Hercules, CA, USA), where the relative expression values were calculated using the Ct comparative method (Vandesompele J, De Preter K, Pattyn F, Poppe B, Van Roy N, De Paepe A, Speleman F. Genome Biol. Jun 18, 2002; 3(7)).

For the RT-qPCR assays performed in melanoma and nevus biopsy samples, histological sections of 10 microns were made from the paraffin blocks for RNA extraction. RNA was purified from the melanoma and nevus biopsies by means of the "RecoverAll Total Nucleic Acid" extraction kit of Ambion. The RNA was quantified and its integrity was determined with Bioanalyzer. For reverse transcription, iScrip cDNA synthesis kit of Bio-Rad was used. The selected genes and the constitutive genes (at least 3 different genes) were re-amplified using Platinum Multiplex PCR Master Mix of Applied Biosystems. Finally, the RT-qPCR is carried out with iQ SYBR Green Supermix of BioRad. The primers used were the same as those used with the melanoma cell lines and melanocytes.

Table 2 shows the primer pairs used in the RT-qPCR assays.

**Table 2**

| Reference genes | | | | |
|---|---|---|---|---|
| Gene | Primer sequence | Size of the amplicon (bp) | | T (°C) Standard curve optimization |
| *β-actin (ACTB)* | Fw 5'-AGATGACCCAGATCATGTTTGAG-3' (SEQ ID NO: 1) | 119 | 60 | r = 0.999 |
| | | | | Ef = 93% |
| | Rev 5'-GTCACCGGAGTCCATCACG-3' (SEQ ID NO: 2) | | | |
| *GAPDH* | Fw 5'-CCTGTTCGACAGTCAGCCG-3' (SEQ ID NO: 3) | 102 | 56 | r = 0.999 |
| | | | | Ef = 95.5% |
| | Rev 5'-CGACCAAATCCGTTGACTCC-3'(SEQ ID NO: 4) | | | |
| *RPS15* | Fw 5'-TTCCGCAAGTTCACCTACC-3' (SEQ ID NO: 5) | 360 | 60 | r = 0.999 |
| | | | | Ef = 90% |
| | Rev 5'-CGGGCCGGCCATGCTTTACG-3' (SEQ ID NO: 6) | | | |
| | | | | |

| Genes under study | | | | |
|---|---|---|---|---|
| Gene | Primer sequence | Size of amplicon (bp) | T (°C) | Standard curve optimization |
| *BCL2* | Fw 5'-GGGGAGGATTGTGGCCTTC-3' (SEQ ID NO: 7) | 94 | 61 | r = 0.998 |
| | | | | Ef = 99.7% |
| | Rev 5'-CAGGGCGATGTTGTCCACC-3' (SEQ ID NO: 8) | | | |
| *BCL3* | Fw 5'-AACCTGCCTACACCCCTATACC-3' (SEQ ID NO: 9) | 120 | 55 | r = 0.997 |
| | | | | Ef = 93% |
| | Rev 5'-GCACCACAGCAATATGGAGAG-3'(SEQ ID NO: 10) | | | |
| *M-MITF* | Fw 5'-CTCGAAAACCCCACCAAGTA-3' (SEQ ID NO: 11) | 115 | 55 | r = 0.992 |
| | | | | Ef = 93% |
| | Rev 5'-GACATGGCAAGCTCAGGACT-3' (SEQ ID NO: 12) | | | |
| *PEBP1* | Fw 5'-AATAGACCCACCAGCATTTCG-3' (SEQ ID NO: 13) | 161 | 61 | r = 0.998 |
| | | | | Ef = 94.5% |
| | Rev 5'-GTGCCACTGCTGATGTCATTG-3' (SEQ ID NO: 14) | | | |
| *PIR* | Fw 5'-GGAGCCTCAGTACCAGGAACT-3' (SEQ ID NO: 15) | 106 | 61 | r = 0.999 |
| | | | | Ef = 97% |
| | Rev 5'-CTTGGACTTTATTCCCAGGGC-3' (SEQ ID NO: 16) | | | |

### Preparation of histological sections and immunohistochemistry

Serial sections 4-6 micron thick were made. The histological sections were processed for immunohistochemistry study using monoclonal antibodies of the selected proteins: PIRIN (ab55978 of Abcam) and BCL3 (1E8, ab49470 of Abcam), negative controls and staining with hematoxylin. The development was carried out with AEC (EnVision G/2 System/AP, rabbit/mouse. Permanent Red of Dako).

### Statistical analysis

The level of statistical significance between the means of the samples was determined through the Student's T test. The p-value was considered statistically significant when p <0.05 and significant limit when p> 0.05 and <0.01.

A main component analysis was conducted with all the data obtained from the analyzed tumor markers. Main component analysis is a mathematical method transforming a set of possibly correlated variables into a smaller set of non-correlated variables referred to as "main components". Therefore, given "n" observations of "p" variables, the objective of the main component analysis is to determine "r" new non-correlated variables referred to as main components representing the greatest possible variability of the original variables. Taking into account the extensive set of variables analyzed in the present project (protein levels and gene expression at transcriptional level), a main component analysis was conducted to make interpreting the set of data easier. How the tumor lines are grouped according to the new main components can thus be determined and whether or not the classification obtained may be useful for clinical diagnosis and/or prognosis can be evaluated.

The statistical analysis was conducted using the SPSS program version 19. To that end, the data obtained from protein quantification by Western blot and from gene expression by RT-qPCR were normalized to generate variables with a mean of 0 and a variance of 1. Next, the factorial axes are rotated to make interpreting the significance of the selected factors easier. One of the most typical methods, and the one used in the present project, is Varimax orthogonal rotation developed by Kaiser. The objective of Varimax rotation is to make the correlation of each of the variables as close as possible to 1 with only one of the factors and as close as possible to 0 with all the others.

### Example 1

### Identification of markers for the diagnosis and prognosis of cutaneous melanoma using cell lines

Figures 1 and 2 show the levels of *PIR* and *PEBP1* mRNAs in melanoma cell lines. Said genes are significantly suppressed in comparison with the expression of said genes in the normal melanocyte cells (the p-values are 0.00084 and 5.11E-08, respectively). In contrast, Figure 3 shows that *BCL3* is overexpressed in melanoma cell lines (p-value = 0.0033).

The results suggest that the transcriptional activity of the *PIR* gene may be used as a marker of the malignant progression of melanoma, since its expression is suppressed in all the assayed melanoma cell lines. Furthermore, the melanoma cell lines are characterized by the suppression of the transcriptional activity of the *PEBP1* gene, together with the overexpression of the *BCL3* gene. Therefore, the study of the transcription levels of these genes can serve as markers differentiating melanoma cells from melanocytes and nevi.

The expression of *M-MITF* (a specific isoform of melanocytes) was analyzed in all the melanoma cell lines and normal melanocytes. This transcription factor contains a basic helix-loop-helix leucine zipper domain involved in melanocyte development and survival. However, a transcriptional suppression of the levels of *M-MITF* was found in 70% of the evaluated melanoma cell lines, whereas the three remaining cell lines expressed similar levels of *M-MITF* as those found in cultures of primary melanocytes (Figure 4).

Therefore, it was concluded that the expression levels of *M-MITF* are high in melanoma cell lines which are in a proliferation period, in which it acts at the same time as a transcription factor for genes involved in cell cycle progression and as a transcriptional suppressor of genes involved in invasion. Meanwhile, the expression levels of *M-MITF* decrease when the tumor cells acquire the capacity to invade (Koludrovic and Davidson, Future Oncol. 2013 Feb; 9(2):235-44).

Despite the evidence showing a decrease in the expression of the *M-MITF* gene in melanoma cells, the expression of this transcription factor is essential for cell survival and proliferation through the regulation of the transcription of different genes, such as *BCL2* (McGill et al. Cell. Jun 14, 2002; 109(6) :707-18) . In fact, in the analyzed melanoma cell lines, the expression of *BCL2* directly correlates with the expression of *M-MITF* (Figure 5). Therefore, like melanocytes, the proliferative melanoma cell lines MEL-HO, MEL-Juso and COLO-800 expressed the *M-MITF* and *BCL2* genes. In contrast, the invasive melanoma cell lines A375, Hs294T, HT144, 1205Lu, WM793B, JSG and RPMI7951 showed a suppressed expression of *M-MITF* and *BCL2* in comparison with normal melanocytes and proliferative melanoma lines (see Figures 4 and 5).

On the other hand, although all the evaluated melanoma cell lines showed levels of *PIR* below the levels found in cultures of primary melanocytes, there is a certain heterogeneity between the tumor cell lines that allows making two groups. Therefore, the cultures of primary melanocytes, referred to by the inventors as "Pir+++", showed a high transcriptional activity of the *PIR* gene. However, among the assayed melanoma cell lines, there was a group with little/no expression of *PIR,* referred to by the inventors as "Pir-", and another group of melanoma cell lines, referred to by the inventors as "Pir+", expressing intermediate levels of *PIR* between Pir- and Pir+++ melanocytes.

As regards the data obtained, the inventors have found that the Pir+ melanoma cell lines showed a higher expression of the *M-MITF* and *BCL2* genes than the Pir- cell lines. This correlation of the expression levels can be explained because both *BCL2* and *PIR* are transcriptional targets of *M-MITF.* Therefore, the MEL-HO, MEL-Juso and COLO-800 melanoma cell lines expressing *M-MITF, BCL2* and *PIR* were assigned a proliferative phenotype, whereas the A375, Hs294T, HT-144, 1205Lu, WM793B, JSG and RPMI7951 melanoma cell lines with low transcriptional activity of the *M-MITF, BCL2* and *PIR* genes were grouped into an invasive phenotype.

The data suggest that the decrease in the expression of M-*MITF, BCL2* and *PIR* in melanoma cells causes the tumor cells to acquire the capacity to migrate and invade.

Table 3 shows a summary of the changes observed in the gene expression in melanoma cell lines in comparison with cultures of primary melanocytes.

**Table 3**

| Gene | Change | No. of melanomas | Student's *T*-test (p*-value*) |
|---|---|---|---|
| *PEBP1* | Decreases | 17/19 | 5.11E-08 |
| *PIR* | Decreases | 19/19 | 0.00084 |
| *M-MITF* | Decreases | 7/10 | 0.011 |
| *BCL2* | Decreases | 7/10 | 0.014 |
| *BCL3* | Increases | 10/10 | 0.0033 |

Table 3 shows by columns: the genes analyzed by means of RT-qPCR, the type of change in the levels of mRNA in the melanoma lines with respect to the melanocytes; the number of melanoma lines with a statistically significant change with respect to the total analyzed lines; and the p-value obtained after performing Student's T test.

Finally, a main component analysis was carried out with the normalized relative expression data corresponding to the *PIR, BCL2, BLC3, PEBP1* and *M-MITF* genes of 6 invasive melanoma cell lines (A375, HS294T, HT144, 1205LU, WM793B and JSG), 3 proliferative melanoma cell lines (MEL-HO, MEL-JUSO and COLO-800) and 3 cultures of primary melanocytes from healthy humans (HEMn-LP, HEMn-MP and HEMn-DP).

The results indicate that the first main component includes 78.7% of the total variance of the experiment (Table 4), whereas the remaining axes contribute to the explained total variance in a rather insignificant manner.

**Table 4**

| Explained total variance | | | | | | |
|---|---|---|---|---|---|---|
| Component | Initial eigenvalues | | | Square sums of the saturations of extraction | | |
| | Total | % of variance | % accumulated | Total | % of variance | % accumulated |
| 1 | 3.935 | 78.700 | 78.700 | 3.935 | 78.700 | 78.700 |
| 2 | 0.531 | 10.613 | 89.313 | | | |
| 3 | 0.406 | 8.129 | 97.443 | | | |
| 4 | 0.092 | 1.831 | 99.274 | | | |
| 5 | 0.036 | 0.726 | 100.000 | | | |

| | | | | | | |
|---|---|---|---|---|---|---|
| Extraction method: Main component analysis. | | | | | | |

The coefficients of each gene for calculating the coordinates on the first component are shown in Table 5.

**Table 5**

| Matrix of coefficients for calculating component scores | |
|---|---|
| | Component |
| | 1 |
| *PIR* | 0.228 |
| *PEBP1* | 0.240 |
| *BCL2* | 0.228 |
| *BCL3* | -0.201 |
| *M-MITF* | 0.228 |

| | |
|---|---|
| Extraction method: Main component analysis. Rotation method: Kaiser Varimax Normalization. Component scores. | |

A system based on the expression of these genes using the matrix of coefficients for this first main axis allows visually classifying the different types of melanocytes (Figure 6).

### Example 2

### Analysis of the expression of PIR in the RNA obtained from nevus and melanoma biopsies by means of RT-qPCR and immunohistochemistry.

### • RT-qPCR

The expression of PIR was analyzed in the RNA obtained from 7 melanoma samples and 12 nevus samples. As can be seen in Figure 7, only one transcript was amplified for all the melanoma and nevus samples. Three replicates were made for each sample.

Figure 8A shows that PIR in the nevi are amplified at less cycles with respect to melanomas, the latter being more heterogeneous in terms of their expression levels. After normalizing the expression data with *GAPDH* it can be observed that melanomas have expression levels of *PIR* that are three times less than the expression levels of nevi. These results are significant (p<0.05) despite the melanoma data deviation (Figure 8B) .

It must be pointed out that there is a significant difference between the expression levels of intradermal nevi (I.N) and melanomas. The tendency of the expression of PIR to decrease as the tumor stage progresses is also observed, stage III (AJCC classification) being invasive since node metastasis occurs (Table 6).

Dysplastic nevi could not be assessed although they also showed lower levels of expression than intradermal nevi (benign).

**Table 6. Expression of PIR in intradermal nevi (I.N), dysplastic nevi (Dysp. N.) and melanomas. In the classification of melanomas by clinical stage, it is observed that the expression levels of PIR gradually decrease as the tumor stage increases.**

| | I.N. | Dysp. N. | Melanoma | Stage I Melanoma | Stage II Melanoma | Stage III Melanoma |
|---|---|---|---|---|---|---|
| No | 10 | 2 | 7 | 2 | 2 | 3 |
| Mean | 16.9 | 4.8 | 5.3 | 8 | 6 | 2.7 |
| Dev. | 7.7 | 5.3 | 3 | 0.03 | 1.2 | 1.6 |

### • Immunohistochemistry

To study of expression of PIRIN protein by means of immunohistochemistry, 8 nevus samples and 7 melanoma samples were analyzed, like in the preceding case without knowing the anatomo-pathological diagnosis. All the samples were processed and observed under the microscope. Figure 9 shows how the expression of PIRIN in cell cytoplasm in the sections of nevus is much greater than that of melanomas.

When the presence of PIRIN in relation to the anatomopathological diagnosis was analyzed, it was concluded that similar to that obtained by means of RT-qPCR, the nevi express more PIRIN protein than the melanomas and the expression in the latter also decreases when the tumor stage progresses.

The results obtained by cross-quantifying (+) the presence of PIRIN (red cytoplasms) in the melanoma and nevus lesions are shown below in Table 7.

**Table 7. Quantification of the expression of the PIRIN protein in melanoma and nevus biopsies. Quantification in arbitrary units (in color crosses).**

| | I.N. | Dysp. N. | Stage IA Melanoma | Stage IB Melanoma | Stage III Melanoma |
|---|---|---|---|---|---|
| No. | 7 | 1 | 2 | 2 | 3 |
| | +++++ | +/- | +++ | ++ | - |

### Example 3

### Differential analysis of the expression of Pirin in nevus and melanoma

The expression of PIR was then analyzed in a histological section of a malignant melanoma on an intradermal nevus. To that end, the anti-Pirin monoclonal antibody was used and developed with AEC (EnVision G/2 System/AP, rabbit/mouse. Permanent Red, Dako). The cell nuclei were contrasted with hematoxylin.

Figure 10 shows two parts in the histological preparation: the part showing more depth corresponds to the melanocytes forming the intradermal nevus (IN) on which the melanoma has appeared (area under the epidermis). It can be clearly seen that the nevus cells have a strong red marking for Pirin compared with the melanoma cells the marking of which is much lighter (Figures 10B and 10C), therefore showing that the expression of PIR in melanoma is less than the expression of PIR in benign nevus. Many cells with melanin are observed in the intermediate area.

Therefore, the expression of PIR can be used as a marker for differentiating a benign nevus from a melanoma.

## Claims

1. An *in vitro* method for diagnosing cutaneous melanoma which comprises:
a) determining the expression level of *PIR (Pirin)* in a sample from a subject, and
b) comparing the level obtained in step a) with a reference value for said marker,
wherein a decreased expression level of *PIR* with respect to the reference value for said marker is indicative that said subject suffers from cutaneous melanoma, wherein the sample is a tissue sample.

2. *The in vitro* method according to claim 1, which further comprises determining the expression level of BCL-3 (B-cell lymphoma 3) and/or PEBP1 (phosphatidylethanolamine-binding protein 1) wherein an increased expression of BCL3 and/or a decreased expression of PEBP1 with respect to the corresponding reference value is indicative that the subject suffers from cutaneous melanoma.

3. An *in vitro* method for determining the probability of metastasis in a subject diagnosed with cutaneous melanoma which comprises:
a) determining the expression level of *PIR* in a sample obtained from a subject, and
b) comparing the level obtained in step a) with a reference value for said marker,
wherein a decreased expression level of *PIR* with respect to the reference value for said marker is indicative that said subject has a high probability of developing metastasis, wherein the sample is a tissue sample.

4. The method according to claim 3, which further comprises determining the expression level of *M-MITF* (Microphthalmia-associated transcription factor) and/or *BCL2* (B-cell lymphoma 2) wherein a decreased expression of M-MTIF and/or a decreased expression of BCL2 with respect to the corresponding reference value is indicative that the subject has a high probability of developing metastasis.

5. An *in vitro* method for differentiating benign nevus from cutaneous melanoma in a cutaneous lesion which comprises:
a) determining the expression level of *PIR* in a tissue sample of said cutaneous lesion, and
b) comparing the level obtained in step a) with a reference value for said marker,
wherein a decreased expression level of *PIR* with respect to the reference value for said marker is indicative that said cutaneous lesion is a melanoma.

6. The method according to claim 5, wherein the benign nevus is dysplastic nevus or Spitz nevus.

7. The method according to any of claims 5 or 6, which further comprises determining the expression level of *BCL3* and/or *PEBP1,* wherein an increased expression of BCL3 and/or a decreased expression of PEBP1 with respect to the corresponding reference value is indicative that the cutaneous lesion is melanoma.

8. The method according to any of claims 1 to 7, wherein the expression level of said marker is determined by means of immunohistochemistry, Western blot, ELISA or a protein array.

9. The method according to any of claims 1 to 7, wherein the expression level of said marker is determined by means of a quantitative polymerase chain reaction (PCR) or by means of a DNA or RNA array or by means of nucleotide hybridization techniques.

10. A kit comprising an antibody or nucleic acid capable of determining the expression level of *PIR* and an antibody or nucleic acid capable of determining the expression level of a marker selected from the group consisting of *BCL3, PEBP1, M-MITF, BCL2* and combinations thereof.

11. The kit according to claim 10, wherein the nucleic acids are coupled to a DNA array or an RNA array.

12. Use of a kit comprising an antibody or a nucleic acid capable of determining the expression level of PIR or use of a kit according to any of claims 10 or 11 for diagnosing cutaneous melanoma, for determining the probability of metastasis in a subject diagnosed with cutaneous melanoma or for differentiating a benign nevus from cutaneous melanoma.

13. Use of a kit comprising an antibody or a nucleic acid capable of determining the expression level of PIR according to claim 12 wherein the nucleic acid is coupled to a DNA array or an RNA array.

## Patentansprüche

1. *In vitro*-Verfahren zum Diagnostizieren von kutanem Melanom, umfassend:
a) Bestimmen des Expressionsspiegels von *PIR (Pirin)* in einer Probe eines Individuums, und
b) Vergleichen des in Schritt a) erhaltenen Spiegels mit einem Referenzwert für den Marker,
wobei ein verringerter Expressionsspiegel von *PIR* in Bezug auf den Referenzwert für den Marker darauf hinweist, dass das Individuum an kutanem Melanom leidet, wobei die Probe eine Gewebeprobe ist.

2. *In vitro*-Verfahren nach Anspruch 1, das des Weiteren das Bestimmen des Expressionsspiegels von BCL-3 (B-Zell-Lymphom 3) und/oder PEBP1 (Phosphatidylethanolamin-bindendes Protein 1) umfasst, wobei eine erhöhte Expression von BCL3 und/oder eine verringerte Expression von PEBP1 in Bezug auf den entsprechenden Referenzwert darauf hinweist, dass das Individuum an kutanem Melanom leidet.

3. *In vitro*-Verfahren zum Bestimmen der Wahrscheinlichkeit von Metastasen in einem Individuum, bei dem kutanes Melanom diagnostiziert wurde, umfassend:
a) Bestimmen des Expressionsspiegels von *PIR* in einer Probe, die von einem Individuum erhalten wurde, und
b) Vergleichen des in Schritt a) erhaltenen Spiegels mit einem Referenzwert für den Marker,
wobei ein verringerter Expressionsspiegel von *PIR* in Bezug auf den Referenzwert für den Marker darauf hinweist, dass das Individuum mit hoher Wahrscheinlichkeit Metastasen entwickelt, wobei die Probe eine Gewebeprobe ist.

4. Verfahren nach Anspruch 3, das des Weiteren das Bestimmen des Expressionsspiegels von *M-MITF* (Mikrophthalmie-assoziierter Transkriptionsfaktor) und/oder *BCL2* (B-Zell-Lymphom 2) umfasst, wobei eine verringerte Expression von M-MTIF und/oder eine verringerte Expression von BCL2 in Bezug auf den entsprechenden Referenzwert darauf hinweist, dass das Individuum mit hoher Wahrscheinlichkeit Metastasen entwickelt.

5. *In vitro*-Verfahren zur Unterscheidung von gutartigem Nävus von kutanem Melanom in einer kutanen Läsion, umfassend:
a) Bestimmen des Expressionsspiegels von *PIR* in einer Gewebeprobe der kutanen Läsion, und
b) Vergleichen des in Schritt a) erhaltenen Spiegels mit einem Referenzwert für den Marker,
wobei ein verringerter Expressionsspiegel von *PIR* in Bezug auf den Referenzwert für den Marker darauf hinweist, dass die kutane Läsion ein Melanom ist.

6. Verfahren nach Anspruch 5, wobei der gutartige Nävus dysplastischer Nävus oder Spitz-Nävus ist.

7. Verfahren nach einem der Ansprüche 5 oder 6, das des Weiteren das Bestimmen des Expressionsspiegels von *BCL3* und/oder *PEBP1* umfasst, wobei eine erhöhte Expression von BCL3 und/oder eine verringerte Expression von PEBP1 in Bezug auf den entsprechenden Referenzwert darauf hinweist, dass die kutane Läsion ein Melanom ist.

8. Verfahren nach einem der Ansprüche 1 bis 7, wobei der Expressionsspiegel des Markers durch Immunhistochemie, Western-Blot, ELISA oder einen Proteinarray bestimmt wird.

9. Verfahren nach einem der Ansprüche 1 bis 7, wobei der Expressionsspiegel des Markers durch eine quantitative Polymerasekettenreaktion (PCR) oder durch einen DNA- oder RNA-Array oder durch Nucleotid-Hybridisierungstechniken bestimmt wird.

10. Kit, umfassend einen Antikörper oder eine Nucleinsäure, der/die in der Lage ist, den Expressionsspiegel von *PIR* zu bestimmen und einen Antikörper oder eine Nucleinsäure, der/die in der Lage ist, den Expressionsspiegel eines Markers zu bestimmen, der ausgewählt ist aus der Gruppe bestehend aus *BCL3, PEBP1, M-MITF, BCL2* und Kombinationen davon.

11. Kit nach Anspruch 10, wobei die Nucleinsäuren an einen DNA-Array oder einen RNA-Array gekoppelt sind.

12. Verwendung eines Kits, umfassend einen Antikörper oder eine Nucleinsäure, der/die in der Lage ist, den Expressionsspiegel von PIR zu bestimmen oder Verwendung eines Kits nach einem der Ansprüche 10 oder 11 zum Diagnostizieren von kutanem Melanom, zur Bestimmung der Wahrscheinlichkeit von Metastasen in einem Individuum, bei dem kutanes Melanom diagnostiziert wurde, oder zur Unterscheidung eines gutartigen Nävus von einem kutanem Melanom.

13. Verwendung eines Kits, umfassend einen Antikörper oder eine Nucleinsäure, der/die in der Lage ist, den Expressionsspiegel von PIR nach Anspruch 12 zu bestimmen, wobei die Nucleinsäure an einen DNA-Array oder einen RNA-Array gekoppelt ist.

## Revendications

1. Une méthode *in vitro* pour diagnostiquer un mélanome cutané qui comprend:
a) le fait de déterminer le niveau d'expression de *PIR (Pirin)* dans un échantillon provenant d'un sujet, et
b) le fait de comparer le niveau obtenu à l'étape a) avec une valeur de référence pour ledit marqueur,
un niveau d'expression réduit de *PIR* par rapport à la valeur de référence pour ledit marqueur étant indicatif du fait que ledit sujet souffre de mélanome cutané, l'échantillon étant un échantillon de tissu.

2. La méthode *in vitro* selon la revendication 1, qui comprend en outre le fait de déterminer le niveau d'expression de BCL-3 (lymphome 3 à cellules B) et/ou de PEBP1 (protéine 1 de liaison à la phosphatidyléthanolamine), une expression accrue de BCL3 et/ou une expression réduite de PEBP1 par rapport à la valeur de référence correspondante étant indicative du fait que le sujet souffre de mélanome cutané.

3. Une méthode *in vitro* pour déterminer la probabilité de métastases chez un sujet diagnostiqué d'un mélanome cutané, qui comprend:
a) le fait de déterminer le niveau d'expression de *PIR* dans un échantillon obtenu d'un sujet, et
b) le fait de comparer le niveau obtenu à l'étape a) avec une valeur de référence pour ledit marqueur,
un niveau d'expression réduit de *PIR* par rapport à la valeur de référence pour ledit marqueur étant indicateur du fait que ledit sujet a une probabilité élevée de développer une métastase, l'échantillon étant un échantillon de tissu.

4. La méthode selon la revendication 3, qui comprend en outre le fait de déterminer le niveau d'expression de *M-MITF* (facteur de transcription associé à la microphtalmie) et/ou de *BCL2* (lymphome 2 à cellules B), une expression réduite de M-MTIF et/ou une expression réduite de BCL2 par rapport à la valeur de référence correspondante étant indicatrice du fait que le sujet a une probabilité élevée de développer une métastase.

5. Une méthode *in vitro* pour différencier un naevus bénin d'un mélanome cutané dans une lésion cutanée, qui comprend:
a) le fait de déterminer le niveau d'expression de *PIR* dans un échantillon de tissu de ladite lésion cutanée, et
b) le fait de comparer le niveau obtenu à l'étape a) avec une valeur de référence pour ledit marqueur,
un niveau d'expression réduit de *PIR* par rapport à la valeur de référence pour ledit marqueur étant indicatif du fait que ladite lésion cutanée est un mélanome.

6. La méthode selon la revendication 5, dans lequel le naevus bénin est un naevus dysplasique ou naevus de Spitz.

7. La méthode selon l'une quelconque des revendications 5 ou 6, qui comprend en outre le fait de déterminer le niveau d'expression de *BCL3* et/ou de *PEBP1,* une expression accrue de BCL3 et/ou une expression réduite de PEBP1 par rapport à la valeur de référence correspondante étant indicatrice du fait que la lésion cutanée est un mélanome.

8. La méthode selon l'une quelconque des revendications 1 à 7, dans lequel le niveau d'expression dudit marqueur est déterminé par immunohistochimie, Western blot, ELISA ou un réseau de protéines.

9. La méthode selon l'une quelconque des revendications 1 à 7, dans lequel le niveau d'expression dudit marqueur est déterminé au moyen d'une réaction en chaîne de la polymérase (PCR) quantitative ou au moyen d'un réseau d'ADN ou d'ARN ou au moyen de techniques d'hybridation nucléotidique.

10. Un kit comprenant un anticorps ou un acide nucléique apte à déterminer le niveau d'expression de *PIR* et un anticorps ou un acide nucléique apte à déterminer le niveau d'expression d'un marqueur choisi dans le groupe constitué par *BCL3, PEBP1, M-MITF, BCL2* et leurs combinaisons.

11. Le kit selon la revendication 10, dans lequel les acides nucléiques sont reliés à un réseau d'ADN ou à un réseau d'ARN.

12. Utilisation d'un kit comprenant un anticorps ou un acide nucléique apte à déterminer le niveau d'expression de *PIR* ou utilisation d'un kit selon l'une quelconque des revendications 10 ou 11 pour le diagnostic du mélanome cutané, pour déterminer la probabilité de métastases chez un sujet diagnostiqué comme ayant un mélanome cutané ou pour différencier un naevus bénin d'un mélanome cutané.

13. Utilisation d'un kit comprenant un anticorps ou un acide nucléique apte à déterminer le niveau d'expression de *PIR* selon la revendication 12, l'acide nucléique étant reliée à un réseau d'ADN ou un réseau d'ARN.
